# EUROPEAN PATENT APPLICATION

(11) **EP 4 154 892 A1**
(43) Date of publication of application: **29.03.2023**
(21) Application number: 21809045.4
(22) Date of filing: 18.05.2021
(51) Int. Cl.: A61K 31/5377, A61P 35/00, C12Q 1/6886

(54) **PHARMACEUTICAL COMPOSITION FOR PREVENTING OR TREATING SMALL-CELL LUNG CANCER ASSOCIATED WITH RON MUTANTS AND METHOD USING SAME**

(30) Priority: 18.05.2020 KR 20200059129
(71) Applicant: Wellmarker Bio Co., Ltd., Seoul 05855 (KR)
(72) Inventor: SON, Hye-Jin, Seoul 05855 (KR); LEE, Mi-So, Seoul 05855 (KR); KIM, Hyo-Jin, Seoul 05855 (KR); YOO, Moon-Seong, Seoul 05855 (KR); LEE, Jun-Hyung, Seoul 05855 (KR); JI, Yu-Geun, Seoul 05855 (KR)
(74) Representative: HGF
(86) International application number: PCT/KR2021/006180
(87) International publication number: WO 2021/235811

(57) **Abstract**

The present invention relates to a pharmaceutical composition for preventing or treating small cell lung cancer associated with a RON mutation and a method using the same. The compound according to the present invention may be usefully used as a precision medicine in the treatment of a patient with small cell lung cancer which has resistance to an EGFR-targeted therapeutic agent used in conventional anticancer therapy and has the RON△155, RON△160, and RON△165 mutations.

## Description

### Technical Field

The present invention relates to a pharmaceutical composition for preventing or treating small cell lung cancer associated with a RON mutation and a method for preventing or treating small cell lung cancer using the same.

### Background Art

Lung cancer refers to a malignant tumor that occurs in the lungs, and can be largely divided into primary lung cancer, in which cancer cells first occur in the bronchi or alveoli, and metastatic lung cancer, in which cancer cells occur in other organs and travel to the lungs via blood vessels or lymphatic vessels to proliferate. Depending on the tissue type, it is largely divided into small cell lung cancer and non-small cell lung cancer, that is, lung cancer other than small cell lung cancer.

Lung cancer, irrespective of tissue type, is most often caused by smoking. In the case of small cell lung cancer, smoking is also the most common cause. Small cell lung cancer accounts for 18% of all lung cancers, but unlike adenocarcinoma, it is an extremely rare tissue type among lung cancers that occur in people who have never smoked. Hemoptysis, wheezing (coarse whistling sound when you breathe), cough, and the like are symptoms that can occur in small cell lung cancer. However, these symptoms are not unique to small cell lung cancer and can also occur in common lung cancer. Since small cell lung cancer occurs frequently in and around the bronchi, chest pain due to invasion of the pleura or chest wall occurs relatively low. In addition, small cell lung cancer, referred to as paraneoplastic syndrome, commonly presents symptoms caused by a kind of hormone-like substance produced by cancer cells. For example, loss of consciousness or systemic weakness may appear due to electrolyte imbalances, and in rare cases, abnormalities in the nerve or muscle system may occur.

It is reported that the 5-year survival rate for lung cancer is about 10%, and the proportion of patients eligible for surgery is 40-50%, the proportion of patients with stage 1 is 60-80%, the proportion of patients with stage 2 is 30-50%, the proportion of patients with stage 3A is 10-30%, the proportion of patients with stage 3B is 5%, and the proportion of patients with stage 4 is 2%. In addition, it should be noted that, in the case of long-term survivors, secondary primary cancer may occur with a frequency of 3 to 5% annually, and the importance of developing therapeutic drugs for this is increasing.

On the other hand, cetuximab is a monoclonal antibody that targets the epidermal growth factor receptor (EGFR). The antibody specifically binds to EGFR on the cell surface to inhibit the proliferation of cancer cells. In particular, since EGFR is overexpressed in cancer cells of metastatic small cell lung cancer, metastatic non-small cell lung cancer, and metastatic colorectal cancer, cetuximab is mainly used for the treatment of the disease.

However, cetuximab cannot be used in patients having resistance to the anticancer agent. There are several causes of resistance to cetuximab. Representatively, resistance to an EGFR-targeted therapeutic agent can be characterized by being associated with a RON (Recepteur d'origine nantais) mutation. Whether or not RON is active plays an important role in tumor development, progression and metastasis. In particular, it has been reported that overexpression or overactivation in lung cancer, colorectal cancer and breast cancer induces tumor infiltration and metastasis and contributes to inhibition of cell death. Substances capable of specifically inhibiting the abnormal activity of RON can effectively treat various diseases associated with RON, particularly tumors such as small cell lung cancer.

RON is a protein receptor belonging to the c-MET family, and is a receptor for a serum protein (macrophage-stimulating protein; MSP) that is secreted by the liver and regulates the action of macrophages. In order to determine the effectiveness of anticancer therapy such as cetuximab, it is known that a mutation in RON significantly improves the response to the drug and survival period in cancer patients, and thus, it is considered very important.

Therefore, in order to effectively treat cancer, there is a need for a new anticancer agent applicable to cancer containing a RON mutation that is resistant to conventional anticancer agents.

### Detailed Description of Invention

### Technical Problem

An object of the present invention is to provide a pharmaceutical composition for preventing or treating small cell lung cancer, comprising a compound capable of preventing or treating small cell lung cancer containing a RON mutation, or a pharmaceutically acceptable salt thereof, as an active ingredient, and a method for preventing or treating small cell lung cancer using the same.

### Solution to Problem

In order to achieve the above object, in one aspect of the present invention, there is provided a pharmaceutical composition for preventing or treating small cell lung cancer, comprising a compound represented by Formula 1 or Formula 2 or a pharmaceutically acceptable salt thereof as an active ingredient: 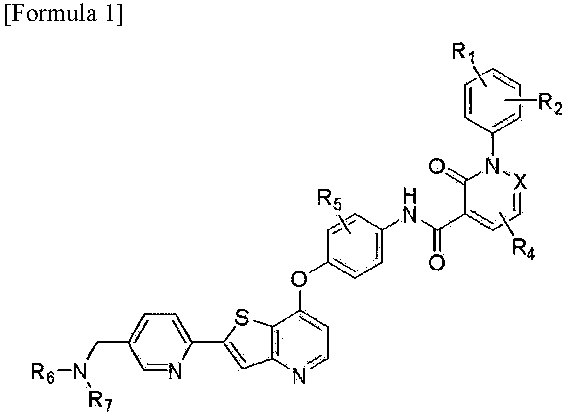 ; and

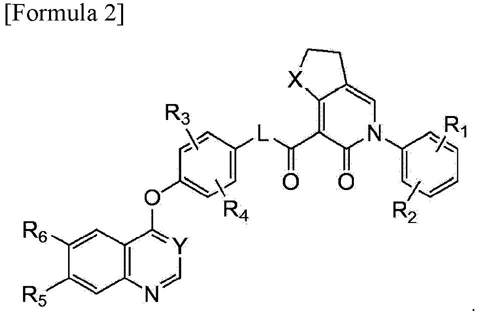

In another aspect of the present invention, there is provided a method for preventing or treating small cell lung cancer, comprising: detecting a mutation in RON in a biological sample derived from a subject suffering from small cell lung cancer, wherein the RON mutation is RONΔ155 in which exons 5, 6 and 11 are deleted, RONΔ160 in which exons 5 and 6 are deleted, or RON△165 in which exon 11 is deleted; and administering the pharmaceutical composition according to claim 1 to a subject in which the mutation in RON is detected.

In another aspect of the present invention, there is provided a method of providing information on an anticancer therapeutic agent, comprising: detecting a mutation in RON in a biological sample derived from a subject suffering from small cell lung cancer, wherein the RON mutation is RON△155 in which exons 5, 6 and 11 are deleted, RONΔ160 in which exons 5 and 6 are deleted, or RON△165 in which exon 11 is deleted; and providing information that the pharmaceutical composition according to claim 1 is suitable for the prevention or treatment of small cell lung cancer to a subject in which the mutation in RON is detected.

In another aspect of the present invention, there is provided the use of the pharmaceutical composition for the prevention or treatment of small cell lung cancer.

### Effects of Invention

The pharmaceutical composition for preventing or treating cancer according to the present invention may be applied to a patient with small cell lung cancer in which a RON mutation is present. In particular, the pharmaceutical composition may be usefully used as a precision medicine in the treatment of a patient with small cell lung cancer which has resistance to cetuximab used in conventional anticancer therapy and has the RON△155, RON△160 and RONΔ165 mutations.

### Brief Description of Drawings

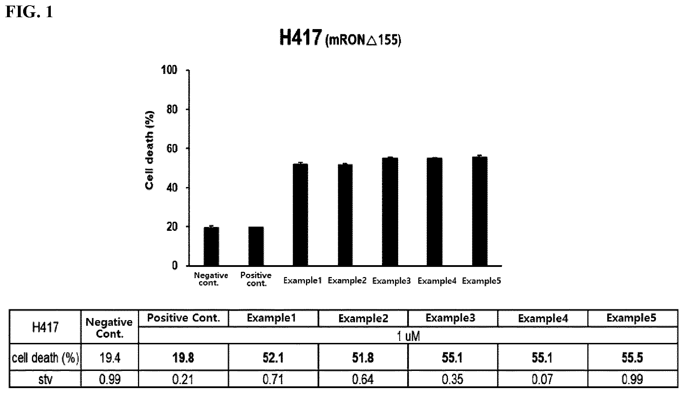

FIG. 1 illustrates the results obtained by comparing and confirming the cell killing efficacy for the H417 cell line, which is a small cell lung cancer cell line of a mutation RONΔ155 type, having been treated with the compounds of Examples 1 (WM-S1-030) to 5 according to the present invention at a concentration of 1 µM, respectively. In this case, BMS-777607, a known RON inhibitor, was used as a positive control.

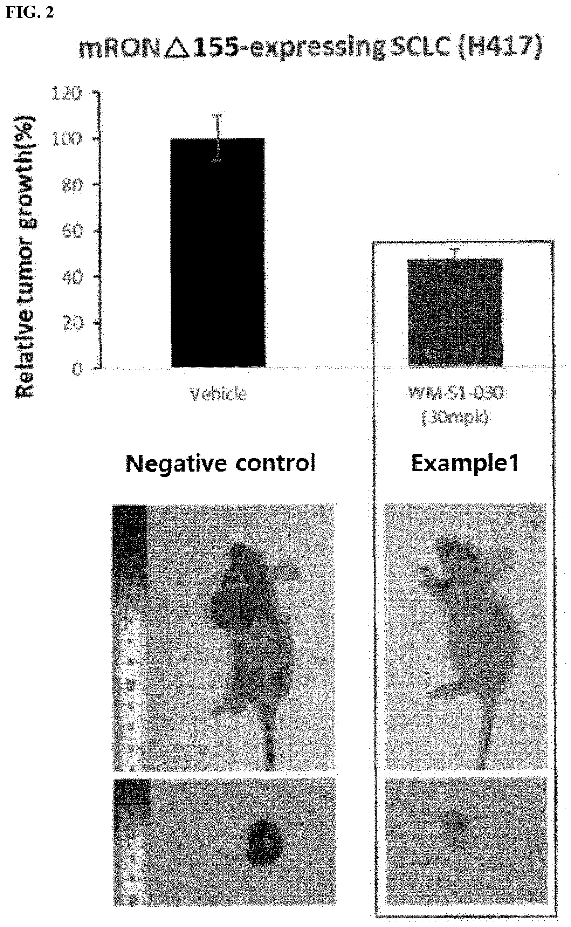

FIG. 2 illustrates the results obtained by confirming the tumor growth rate for an animal model transplanted with the H417 cell line, which is a small cell lung cancer cell line of a mutation RONΔ155 type, having received the compound WM-S 1-030 (Example 1) according to the present invention at a dose of 30 mpk. Specifically, it illustrates (top) a graph showing the relative tumor growth rate derived by comparing a negative control orally administered with only the vehicle and an experimental group orally administered with the compound of Example 1; and (bottom) a photograph of the extracted tumor.

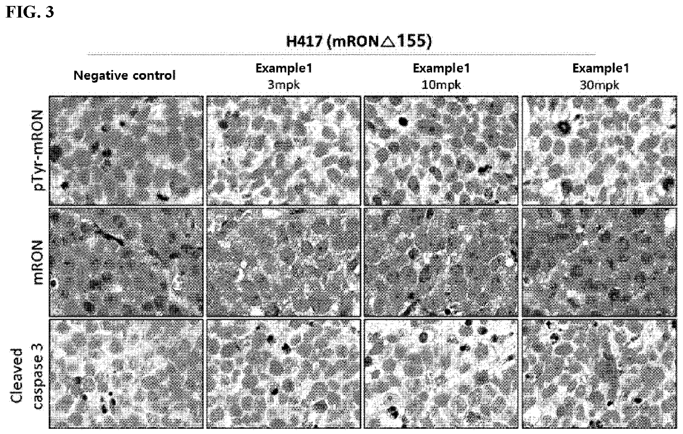

FIG. 3 illustrates the results obtained by confirming the expression level of pTyr-mRON and cleaved caspase-3 by immunochemical staining of the tumor tissue for an animal model transplanted with the H417 cell line, which is a small cell lung cancer cell line of a mutation RONΔ155 type, having received the compound WM-S 1-030 (Example 1) according to the present invention at a dose of 3 mpk, 10 mpk and 30 mpk.

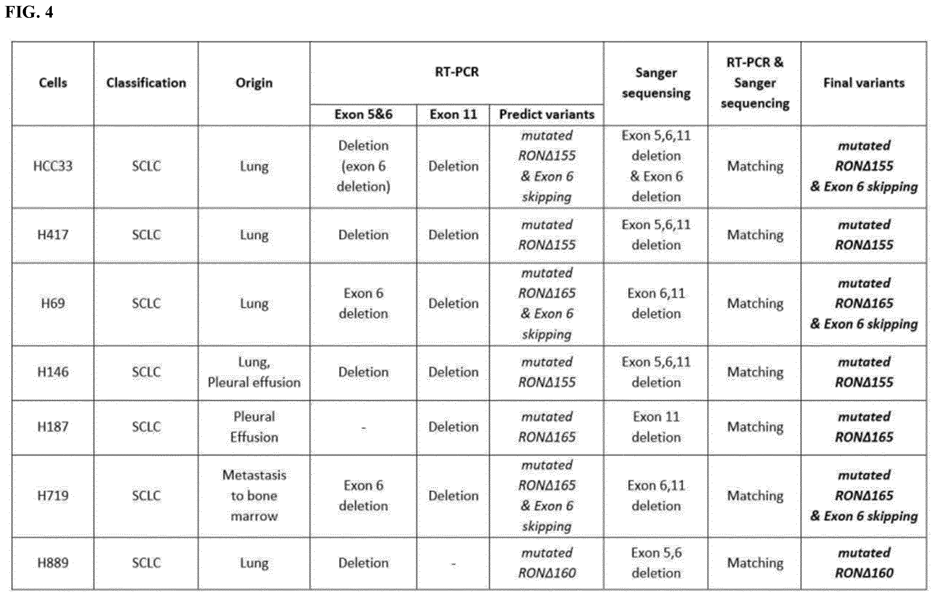

FIG. 4 is a table showing whether a RON mutation is present by analyzing the RON mutation sequences in seven small cell lung cancer cell lines, HCC33, H417, H69, H146, H187, H719 and H889.

### Best Mode for Carrying out the Invention

Hereinafter, the present invention will be described in more detail.

In one aspect of the present invention, there is provided a pharmaceutical composition for preventing or treating small cell lung cancer, comprising a compound represented by Formula 1 or Formula 2 or a pharmaceutically acceptable salt thereof as an active ingredient.

In this case, the small cell lung cancer may be one in which a RON (Recepteur d'origine nantais) mutation is present. In particular, in the present specification, the small cell lung cancer may have resistance to an EGFR-targeted therapeutic agent. In addition, the EGFR-targeted therapeutic agent may be at least one selected from the group consisting of cetuximab, gefitinib, erlotinib, apatinib, icotinib, brigatinib, lapatinib, canertinib, AEE788, XL647, zactima, and panitumumab.

As used herein, the term "RON" is a protein encoded by the human MST1R (macrophage stimulating 1 receptor) gene. It is a protein receptor belonging to the c-MET family, and it is a receptor for a serum protein (macrophage-stimulating protein; MSP) that is secreted by the liver and regulates the action of macrophages. Ligand binding at the cell surface induces the phosphorylation of RON in the intracellular domain, thereby providing a docking site for downstream signaling molecules. Signals from RON activate the wound healing response by promoting epithelial cell migration, proliferation, and survival at the wound site. It plays a role in the innate immune response by regulating macrophage migration and phagocytic activity. In addition, RON may also promote signals such as cell migration and proliferation in response to growth factors other than MST1 ligands. RON is mainly expressed in epithelial cells of liver, lung, intestine, kidney, brain, bone, adrenal gland, skin, and the like.

The mutated form of the RON is found in various solid cancers such as small cell lung cancer (SCLC), non-small cell lung cancer (NSCLC), pancreatic cancer, and colorectal cancer. In this case, the RON may have the nucleotide sequence of SEQ ID NO: 2, 3 or 4.

As used herein, the term "MST1R gene" refers to a tyrosine kinase receptor that transduces a signal to the cytoplasm by binding to the MST1 ligand. It regulates a variety of physiological processes, including cell survival, migration, and differentiation.

As used herein, the term "RON mutation" may be characterized in that it is at least one of RONΔ155 in which exons 5, 6 and 11 are deleted, RONΔ160 in which exons 5 and 6 are deleted, and RONΔ165 in which exon 11 is deleted. The cDNA of RONΔ155 may have the nucleotide sequence of SEQ ID NO: 2. The cDNA of RONΔ160 may have the nucleotide sequence of SEQ ID NO: 3. The cDNA of RONΔ165 may have the nucleotide sequence of SEQ ID NO: 4.

As used herein, the term "resistance" means that there is no efficacy of the drug because it does not react sensitively to the drug.

As used herein, the term "EGFR-targeted therapeutic agent" refers to an anticancer agent that targets EGFR, and any EGFR-targeted therapeutic agent may be applied as long as it exhibits anticancer effect. The EGFR-targeted therapeutic agent may be preferably cetuximab, gefitinib, erlotinib, apatinib, icotinib, brigatinib, lapatinib, canertinib, AEE788, XL647, zactima or panitumumab, and most preferably cetuximab.

The compound of Formula 1 used in the present invention is represented as follows: 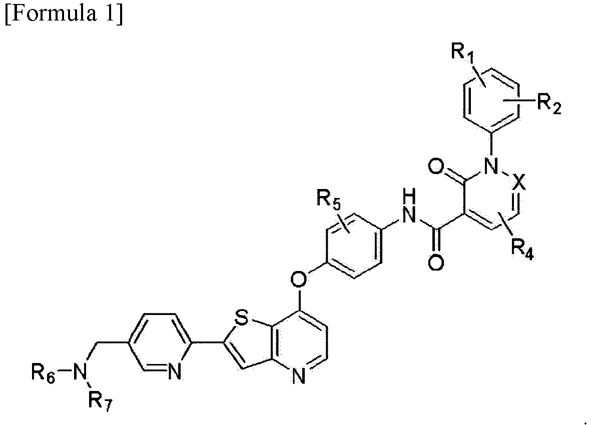 In Formula 1,
R₁ and R₂ are each independently H, halogen, C₁₋₁₀ alkoxy or haloC₁₋₁₀ alkyl;
X is -C(-R₃)= or -N=;
R₃ and R₄ are each independently H, halogen, C₁₋₁₀ alkyl, or C₁₋₁₀ alkoxy;
R₅ is H, halogen, or C₁₋₁₀ alkyl;
R₆ and R₇ are taken together with the N atom to which they are attached to form a 4 to 10-membered heterocycle, or R₆ is -C₂H₄-O-CH₃ and R₇ is H, methyl or t-butoxycarbonyl; and
the heterocycle further has or does not have 1 or 2 heteroatoms selected from the group consisting of N, O and S in addition to the N atom to which R₆ and R₇ are attached, and the heterocycle is also unsubstituted or substituted with at least one selected from halogen and C₁₋₆ alkyl.

The C₁₋₁₀ alkyl may include C₁₋₆ alkyl, C₁₋₃ alkyl, C₃₋₁₀ alkyl, C₃₋₆ alkyl, C₆₋₁₀ alkyl, and the like. In addition, the C₁₋₁₀ alkoxy may include C₁₋₆ alkoxy, C₁₋₃ alkoxy, C₃₋₁₀ alkoxy, C₃₋₆ alkoxy, C₆₋₁₀ alkoxy, and the like. In addition, the 4 to 10-membered heterocycle may include a 4 to 7-membered heterocycle, a 4 to 6-membered heterocycle, a 5 to 7-membered heterocycle, a 5 or 6-membered heterocycle, and the like.

According to one embodiment, in Formula 1, R₁ and R₂ may be each independently H, halogen, methoxy, or -CF₃, wherein halogen may be F, Cl, Br or I.

According to another embodiment, in Formula 1, R₃ and R₄ may be each independently H, halogen, methyl, methoxy, or ethoxy, wherein halogen may be F, Cl, Br or I.

According to another embodiment, in Formula 1, X is -C(-R₃)=; and R₃ and R₄ are each independently H, halogen, methyl, methoxy, or ethoxy, provided that R₃ and R₄ are not both H.

According to another embodiment, in Formula 1, X may be -N=; and R₄ may be halogen, methyl, methoxy, or ethoxy, wherein halogen may be F, Cl, Br or I.

According to another embodiment, in Formula 1, R₅ may be H or halogen, wherein halogen may be F, Cl, Br or I.

According to another embodiment, in Formula 1, R₆ and R₇ may be taken together with the N atom to which they are attached to form 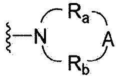 ; wherein Rₐ and R_{b} may be each independently C₁₋₃ alkylene; A may be -N(-R₉)- or -O-, and R₉ may be C₁₋₆ alkyl. As a specific example, R₆ and R₇ may be taken together with the N atom to which they are attached to form azetidinyl, diazetidinyl, pyrrolidinyl, pyrrolyl, imidazolidinyl, imidazolyl, pyrazolidinyl, pyrazolyl, oxazolidinyl, oxazolyl, isoxazolidinyl, isoxazolyl, thiazolidinyl, thiazolyl, isothiazolidinyl, isothiazolyl, piperidinyl, pyridinyl, piperazinyl, diazinyl, morpholino, thiomorpholino, azepanyl, diazepanyl, or a heterocycle group substituted with C₁₋₆ alkyl thereon. In addition, Rₐ and R_{b} may be each independently -CH₂-, -C₂H₄- or -C₃H₆-. In addition, R₉ may be methyl, ethyl, n-propyl, i-propyl, n-butyl, i-butyl, t-butyl, n-pentyl, i-pentyl, t-pentyl, sec-pentyl, neopentyl, hexyl, and the like.

According to another embodiment, in Formula 1, R₁ and R₂ may be each independently H, halogen, methoxy, or -CF₃; R₃ and R₄ may be each independently H, halogen, methyl, methoxy, or ethoxy; R₅ may be H or halogen; and R₆ may be -C₂H₄-O-CH₃ and R₇ may be H, methyl or t-butoxycarbonyl, or R₆ and R₇ may be linked to each other to form morpholino or methylpiperazinyl, wherein halogen may be F, Cl, Br or I.

According to a specific embodiment, the compound of Formula 1 may be represented by Formula 1a below: 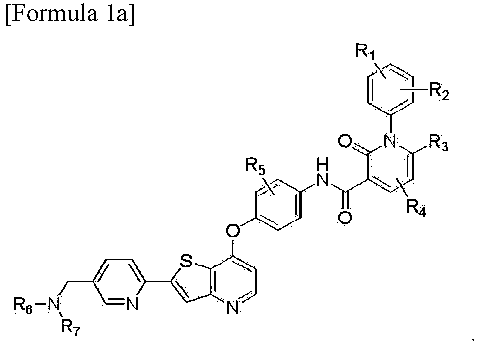 In Formula 1a, R₁ to R₇ are as defined in Formula 1 above.

Specifically, in Formula 1a, R₁ and R₂ may be each independently H, halogen, or -CF₃. In addition, R₃ and R₄ may be each independently H, halogen, methyl, methoxy, or ethoxy, provided that R₃ and R₄ are not both H. In addition, R₅ may be H or halogen.

More specifically, in Formula 1a, R₁ and R₂ may be each independently H, halogen, or - CF₃; R₃ and R₄ may be each independently H, halogen, methyl, methoxy, or ethoxy; R₅ may be H or halogen; and R₆ may be -C₂H₄-O-CH₃, and R₇ may be H, methyl or t-butoxycarbonyl.

According to another specific embodiment, the compound of Formula 1 may be represented by Formula 1b below: 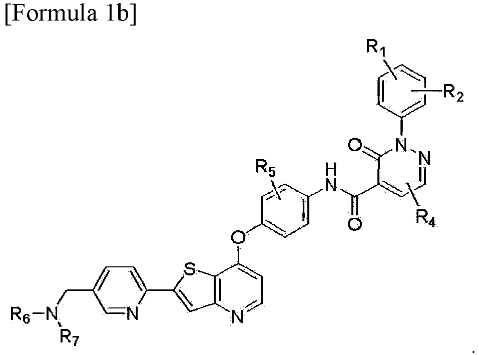 In Formula 1b, R₁ to R₇ are as defined in Formula 1 above.

Specifically, in Formula 1b, R₁ and R₂ may be each independently H, halogen, or -CF₃. In addition, R₄ may be halogen, methyl, methoxy, or ethoxy. In addition, R₅ may be H or halogen.

More specifically, in Formula 1b, R₁ and R₂ may be each independently H, halogen, or - CF₃; R₄ may be halogen, methyl, methoxy, or ethoxy; R₅ may be H or halogen; R₆ may be - C₂H₄-O-CH₃; and R₇ may be H, methyl or t-butoxycarbonyl.

According to another specific embodiment, the compound of Formula 1 may be represented by Formula 1c below: 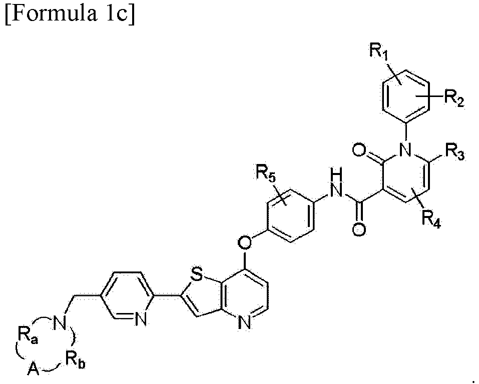 In Formula 1c, R₁ to R₅ may be as defined in Formula 1 above; Rₐ and R_{b} may be each independently C₁₋₃ alkylene; A may be -N(-R₉)- or -O-, and R₉ may be C₁₋₆ alkyl.

Specifically, in Formula 1c, R₁ and R₂ may be each independently H, halogen, or -CF₃. In addition, R₃ and R₄ may be each independently H, halogen, methyl, methoxy, or ethoxy, provided that R₃ and R₄ are not both H. In addition, R₅ may be H or halogen. In addition, Rₐ and R_{b} may be taken together with N and A to which they are attached to form morpholino or methylpiperazinyl.

According to another specific embodiment, the compound of Formula 1 may be represented by Formula 1d below. 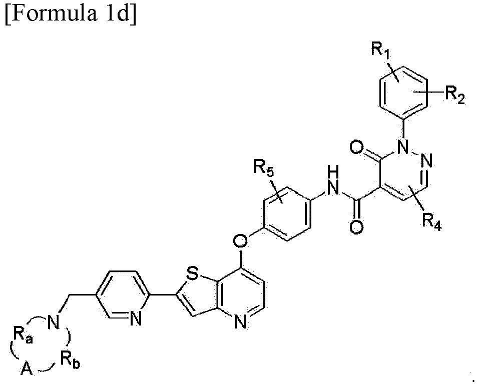 In Formula 1d, R₁ to R₅ may be as defined in Formula 1 above; Rₐ and R_{b} may be each independently C₁₋₃ alkylene; A may be -N(-R₉)- or -O-, and R₉ may be C₁₋₆ alkyl.

Specifically, in Formula 1d, R₁ and R₂ may be each independently H, halogen, or -CF₃. In addition, R₄ may be halogen, methyl, methoxy, or ethoxy. In addition, R₅ may be H or halogen. In addition, Rₐ and R_{b} may be taken together with N and A to which they are attached to form morpholino or methylpiperazinyl.

According to specific embodiments, specific examples of the compound represented by Formula 1 are as follows:
1) 4-ethoxy-N-(3-fluoro-4-{[2-(5-{[(2-methoxyethyl)amino]methyl}pyridin-2-yl)thieno[3,2-*b*]pyridin-7-yl]oxy}phenyl)-2-oxo-1-phenyl-1,2-dihydropyridine-3-carboxamide;
2) N-(3-fluoro-4-{[2-(5-{[(2-methoxyethyl)amino]methyl}pyridin-2-yl)thieno[3,2-*b*]pyridin-7-yl]oxy}phenyl)-1-(4-fluorophenyl)-4-methoxy-2-oxo-1,2-dihydropyridine-3-carboxamide;
3) N-(3-fluoro-4-{[2-(5-{[(2-methoxyethyl)amino]methyl}pyridin-2-yl)thieno[3,2-*b*]pyridin-7-yl]oxy}phenyl)-4-methoxy-2-oxo-1-phenyl-1,2-dihydropyridine-3-carboxamide;
4) N-(3-fluoro-4-{[2-(5-{[(2-methoxyethyl)amino]methyl}pyridin-2-yl)thieno[3,2-*b*]pyridin-7-yl]oxy}phenyl)-1-(4-fluorophenyl)-2-oxo-1,2-dihydropyridine-3-carboxamide;
5) N-(3-fluoro-4-{[2-(5-{[(2-methoxyethyl)amino]methyl}pyridin-2-yl)thieno[3,2-*b*]pyridin-7-yl]oxy}phenyl)-2-oxo-1-phenyl-1,2-dihydropyridine-3-carboxamide;
6) *t*-butyl {[6-(7-{4-[4-ethoxy-1-(4-fluorophenyl)-2-oxo-1,2-dihydropyridine-3-carboxamido]-2-fluorophenoxy}thieno[3,2-*b*]pyridin-2-yl)pyridin-3-yl]methyl}(2-methoxyethyl)carbamate;
7) 4-ethoxy-N-(3-fluoro-4-{[2-(5-{[(2-methoxyethyl)amino]methyl}pyridin-2-yl)thieno[3,2-*b*]pyridin-7-yl]oxy}phenyl)-1-(4-fluorophenyl)-2-oxo-1,2-dihydropyridine-3-carboxamide;
8) 1-(4-chlorophenyl)-4-ethoxy-N-(3-fluoro-4-{[2-(5-{[(2-methoxyethyl)amino]methyl}pyridin-2-yl)thieno[3,2-b]pyridin-7-yl]oxy}phenyl)-2-oxo-1,2-dihydropyridine-3-carboxamide;
9) N-(3-chloro-4-{[2-(5-{[(2-methoxyethyl)amino]methyl}pyridin-2-yl)thieno[3,2-*b*]pyridin-7-yl]oxy}phenyl)-1-(4-fluorophenyl)-4-methoxy-2-oxo-1,2-dihydropyridine-3-carboxamide;
10) N-(2-chloro-4-{[-2-(5-{[(2-methoxyethyl)amino]methyl}pyridin-2-yl)thieno[3,2-*b*]pyridin-7-yl]oxy}phenyl)-1-(4-fluorophenyl)-4-methoxy-2-oxo-1,2-dihydropyridine-3-carboxamide;
11) 1-(4-fluorophenyl)-4-methoxy-N-(4-{[2-(5-{[(2-methoxyethyl)amino]methyl}pyridin-2-yl)thieno[3,2-*b*]pyridin-7-yl)oxy)phenyl)-2-oxo-1,2-dihydropyridine-3-carboxamide;
12) 4-ethoxy-N-(3 -fluoro-4-{[2-(5-{[(2-methoxyethyl)amino]methyl}pyridin-2-yl)thieno[3,2-*b*]pyridin-7-yl]oxy}phenyl)-2-oxo-1-(4-(trifluoromethyl)phenyl)-1,2-dihydropyridine-3-carboxamide;
13) 1-(4-chlorophenyl)-N-(3-fluoro-4-{[2-(5-{[(2-methoxyethyl)amino]methyl}pyridin-2-yl)thieno[3,2-*b*]pyridin-7-yl]oxy}phenyl)-4-methoxy-2-oxo-1,2-dihydropyridine-3-carboxamide;
14) 4-ethoxy-N-(3-fluoro-4-{[2-(5-{[(2-methoxyethyl)amino]methyl}pyri din-2-yl)thieno[3,2-*b*]pyridin-7-yl]oxy}phenyl)-1-(3-fluorophenyl)-2-oxo-1,2-dihydropyridine-3-carboxamide;
15) 4-ethoxy-N-(3-fluoro-4-{[2-(5-{[(2-methoxyethyl)amino]methyl}pyridin-2-yl)thieno[3,2-*b*]pyridin-7-yl]oxy}phenyl)-1-(4-methoxyphenyl)-2-oxo-1,2-dihydropyridine-3-carboxamide;
16) 4-ethoxy-N-(3-fluoro-4-{[2-(5-{[(2-methoxyethyl)amino]methyl}pyridin-2-yl)thieno[3,2-*b*]pyridin-7-yl]oxy}phenyl)-1-(3-methoxyphenyl)-2-oxo-1,2-dihydropyridine-3-carboxamide;
17) N-(3-fluoro-4-{[2-(5-{[(2-methoxyethyl)amino]methyl}pyridin-2-yl)thieno[3,2-*b*]pyridin-7-yl]oxy}phenyl)-2-(4-fluorophenyl)-5-methyl-3-oxo-2,3-dihydropyridazine-4-carboxamide;
18) N-(3-fluoro-4-{[2-(5-{[(2-methoxyethyl)amino]methyl}pyridin-2-yl)thieno[3,2-*b*]pyridin-7-yl]oxy}phenyl)-5-methyl-3-oxo-2-phenyl-2,3-dihydropyridazine-4-carboxamide;
19) N-(3-fluoro-4-{ [2-(5-{ [(2-methoxyethyl)amino]methyl}pyridin-2-yl)thieno[3,2-*b*]pyridin-7-yl]oxy}phenyl)-3-oxo-2-phenyl-2,3-dihydropyridazine-4-carboxamide;
20) N-(3-fluoro-4-[{2-(5-[{(2-methoxyethyl)amino}methyl]pyridin-2-yl)thieno[3,2-*b*]pyridin-7-yl}oxy]phenyl)-2-(4-fluorophenyl)-3-oxo-2,3-dihydropyridazine-4-carboxamide;
21) N-(3-fluoro-4-{[2-(5-{[(2-methoxyethyl)amino]methyl}pyridin-2-yl)thieno[3,2-*b*]pyridin-7-yl]oxy}phenyl)-6-methyl-2-oxo-1-phenyl-1,2-dihydropyridine-3-carboxamide;
22) N-(3-fluoro-4-{[2-(5-{[(2-methoxyethyl)amino]methyl}pyridin-2-yl)thieno[3,2-*b*]pyridin-7-yl]oxy}phenyl)-1-(4-fluorophenyl)-6-methyl-2-oxo-1,2-dihydropyridine-3-carboxamide;
23) 5-bromo-N-(3-fluoro-4-{[2-(5-{[(2-methoxyethyl)amino]methyl}pyridin-2-yl)thieno[3,2-*b*]pyridin-7-yl]oxy}phenyl)-1-(4-fluorophenyl)-2-oxo-1,2-dihydropyridine-3-carboxamide;
24) 5-chloro-N-(3-fluoro-4-{[2-(5-{[(2-methoxyethyl)amino]methyl}pyridin-2-yl)thieno[3,2-*b*]pyridin-7-yl]oxy}phenyl)-1-(4-fluorophenyl)-2-oxo-1,2-dihydropyridine-3-carboxamide;
25) N-(3-fluoro-4-{[2-(5-{[(2-methoxyethyl)amino]methyl}pyridin-2-yl)thieno[3,2-*b*]pyridin-7-yl]oxy}phenyl)-1-(4-fluorophenyl)-4-methyl-2-oxo-1,2-dihydropyridine-3-carboxamide;
26) N-(2-chloro-4-{[2-(5-{[(2-methoxyethyl)amino]methyl}pyridin-2-yl)thieno[3,2-*b*]pyridin-7-yl]oxy}phenyl)-4-ethoxy-1-(4-fluorophenyl)-2-oxo-1,2-dihydropyridine-3-carboxamide;
27) N-(3-fluoro-4-([2-(5-{[(2-methoxyethyl)amino)methyl]pyridin-2-yl}thieno[3,2-*b*]pyridin-7-yl)oxy]phenyl}-1-(4-fluorophenyl)-5,6-dimethyl-2-oxo-1,2-dihydropyridine-3-carboxamide;
28) N-(3-fluoro-4-{[-2-(5-{[(2-methoxyethyl)amino]methyl}pyridin-2-yl)thieno[3,2-*b*]pyridin-7-yl]oxy}phenyl)-4-methyl-2-oxo-1-phenyl-1,2-dihydropyridine-3-carboxamide;
29) N-(3-fluoro-4-{[-2-(5-{[(2-methoxyethyl)amino]methyl}pyridin-2-yl)thieno[3,2-*b*]pyridin-7-yl]oxy}phenyl)-1-(4-fluorophenyl)-5-methyl-2-oxo-1,2-dihydropyridine-3-carboxamide;
30) 4-ethoxy-N-(3-fluoro-4-{[2-(5-{[(2-methoxyethyl)(methyl)amino]methyl}pyridin-2-yl)thieno[3,2-*b*]pyridin-7-yl]oxy}phenyl)-1-(4-fluorophenyl)-2-oxo-1,2-dihydropyridine-3-carboxamide;
31) 4-ethoxy-N-[3-fluoro-4-({2-[5-(morpholinomethyl)pyridin-2-yl]thieno[3,2-*b*]yridin-7-yl}oxy)phenyl]-1-(4-fluorophenyl)-2-oxo-1,2-dihydropyridine-3-carboxamide;
32) 4-ethoxy-N-[3-fluoro-4-({2-[5-(morpholinomethyl)pyridin-2-yl]thieno[3,2-*b*]pyridin-7-yl}oxy)phenyl]-2-oxo-1-phenyl-1,2-dihydropyridine-3-carboxamide;
33) 4-ethoxy-N-{3-fluoro-4-[(2-{5-[(4-methylpiperazin-1-yl)methyl]pyridin-2-yl}thieno[3,2-*b*]pyridin-7-yl)oxy]phenyl}-1-(4-fluorophenyl)-2-oxo-1,2-dihydropyridine-3-carboxamide;
34) 4-ethoxy-N-{3-fluoro-4-[(2-{5-[(-methylpiperazin-1-yl)methyl]pyridin-2-yl}thieno[3,2-*b*]pyridin-7-yl)oxy]phenyl}-2-oxo-1-phenyl-1,2-dihydropyridine-3-carboxamide;
35) 1-(4-chlorophenyl)-4-ethoxy-N-[3-fluoro-4-({2-[5-(morpholinomethyl)pyridin-2-yl]thieno[3,2-*b*]pyridin-7-yl}oxy)phenyl]-2-oxo-1,2-dihydropyridine-3-carboxamide;
36) N-[3-chloro-4-({2-[5-(morpholinomethyl)pyridin-2-yl]thieno[3,2-*b*]pyridin-7-yl}oxy)phenyl]-4-ethoxy-1-(4-fluorophenyl)-2-oxo-1,2-dihydropyridine-3-carboxamide; and
37) N-[2-chloro-4-({2-[5-(morpholinomethyl)pyridin-2-yl]thieno[3,2-*b*]pyridin-7-yl}oxy)phenyl]-4-ethoxy-1-(4-fluorophenyl)-2-oxo-1,2-dihydropyridine-3-carboxamide.

Preferably, the compound represented by Formula 1 may be a compound selected from the group consisting of:
4-ethoxy-N-[3-fluoro-4-({2-[5-(morpholinomethyl)pyridin-2-yl]thieno[3,2-*b*]pyridin-7-yl}oxy)phenyl]-1-(4-fluorophenyl)-2-oxo-1,2-dihydropyridine-3-carboxamide;
4-ethoxy-N-(3-fluoro-4-{ [2-(5-{ [(2-methoxy ethyl)amino]methyl}pyri din-2-yl)thieno[3,2-*b*]pyridin-7-yl]oxy}phenyl)-2-oxo-1-phenyl-1,2-dihydropyridine-3-carboxamide; and
4-ethoxy-N-{3-fluoro-4-[(2-{5-[(-methylpiperazin-1-yl)methyl]pyridin-2-yl}thieno[3,2-*b*]pyridin-7-yl)oxy]phenyl}-2-oxo-1-phenyl-1,2-dihydropyridine-3-carboxamide.

The compound of Formula 1 used in the composition according to the present invention may be prepared by the method disclosed in Korean Patent No. 10-2221689, and may be prepared by various methods based on other known methods and/or techniques in the field of organic synthesis. Based on the above methods, various derivatives may be synthesized using an appropriate synthesis method depending on the type of the substituent.

The compound of Formula 2 used in the present invention is represented as follows: 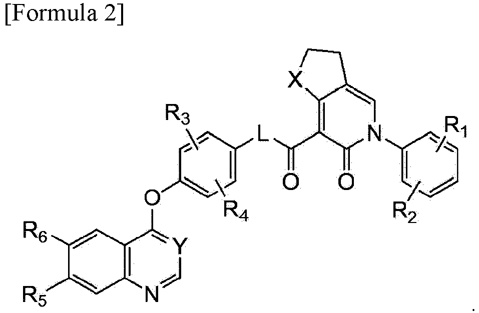 In Formula 2,
L is -NH- or -CH₂-,
R₁ to R₄ are each independently hydrogen, halogen, hydroxy, cyano, C₁₋₄ alkyl, C₁₋₄ alkoxy, C₁₋₄ haloalkyl, C₂₋₄ alkenyl, C₂₋₄ alkynyl, C₃₋₇ cycloalkyl, C₆₋₁₀ aryl, 5- to 9-membered heteroaryl or 3- to 9-membered heterocycloalkyl,
X is O, S, -CH(-Rx)- or -N(-Rx)-,
Rx is hydrogen, C₁₋₄ alkyl, C₁₋₄ alkoxy, C₁₋₄ haloalkyl, C₂₋₄ alkenyl, C₂₋₄ alkynyl, C₆₋₁₀ aryl, C₆₋₁₀ aryl-C₁₋₄ alkyl, or 3- to 9-membered heterocycloalkyl,
Y is -N= or -CH=, and
R₅ and R₆ are each independently hydrogen, amino, halogen, cyano, C₁₋₆ alkyl, C₁₋₆ haloalkyl, C₁₋₆ alkoxy, amino-C₁-₆ alkoxy, aminocarbonyl, C₁₋₆ alkylaminocarbonyl, di-C₁-₆ alkylcarbonylamino, C₁₋₆ alkylcarbonylamino, C₁₋₆ alkylamino, or C₁₋₆ alkyl-amino-C₁-₆ alkoxy,
wherein R₅ and R₆ are each independently unsubstituted or substituted with 3- to 9-membered cycloalkyl; or 3- to 9-membered heterocycloalkyl,
the cycloalkyl or heterocycloalkyl has or does not have at least one substituent selected from the group consisting of halogen, oxo, cyano, hydroxy, hydroxy-C₁-₆ alkyl, amino, di-C₁-₆ alkylamino, C₁₋₆ alkyl, C₁₋₆ alkoxy, and C₁₋₆ alkoxy-C₁-₆ alkyl, and
the heterocycloalkyl contains 1 to 4 heteroatoms selected from the group consisting of N, O and S.

The C₁₋₆ alkyl may include C₁₋₃ alkyl, C₃₋₆ alkyl, and the like. In addition, the C₁₋₆ alkoxy may include C₁₋₃ alkoxy, C₃₋₆ alkoxy, and the like.

According to one embodiment, in Formula 2, R₁ to R₄ may be each independently hydrogen, C₁₋₄ haloalkyl, or halogen, wherein halogen may be F, Cl, Br or I. Specifically, R₁ may be hydrogen, trifluoromethyl, or fluoro, R₂ may be hydrogen, R₃ may be fluoro, and R₄ may be hydrogen.

According to another embodiment, in Formula 2, X may be O or -CH(-Rx)-, and Rx may be hydrogen or C₁₋₆ alkyl.

According to another embodiment, in Formula 2, R₅ and R₆ may be each independently hydrogen, amino, halogen, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, di-C₁₋₆ alkylcarbonylamino, C₁₋₆ alkylcarbonylamino, C₁₋₆ alkylamino, C₁₋₆ alkyl-amino-C₁₋₆ alkoxy, or 5- to 9-membered heteroaryl, wherein R₅ and R₆ may be each independently unsubstituted or substituted with C₁₋₆ alkyl; C₁₋₆ alkyl or C₁₋₆ alkyl-amino-C₁₋₆ alkyl substituted with any one of C₁₋₆ alkoxy-C₁₋₆ alkyl-amino, 3- to 9-membered cycloalkyl and 3- to 9-membered heterocycloalkyl; 3- to 9-membered cycloalkyl; or 3- to 9-membered heterocycloalkyl, wherein the cycloalkyl or heterocycloalkyl may have or may not have at least one substituent selected from the group consisting of halogen, oxo, cyano, hydroxy, hydroxy-C₁₋₆ alkyl, amino, di-C₁₋₆ alkylamino, C₁₋₆ alkyl, C₁₋₆ alkoxy, and C₁₋₆ alkoxy-C₁₋₆ alkyl, and the heteroaryl and the heterocycloalkyl may each independently contain at least one heteroatom selected from the group consisting of N, O and S.

Specifically, the heteroaryl may be pyridinyl, imidazolyl, or pyrazolyl, the heterocycloalkyl may be azetidinyl, pyrrolidinyl, tetrahydropyranyl, morpholino, morpholinyl, dioxidothiomorpholino, piperazinyl, piperidinyl, or oxetanyl, and the cycloalkyl may be cyclobutyl, cyclopentyl, or cyclohexyl.

In addition, when the heteroaryl or the heterocycloalkyl contains at least one N atom, it may be substituted at a position of any one N atom of them, but is not particularly limited.

According to another embodiment, in Formula 2, R₁ and R₂ may be hydrogen, C₁₋₄ haloalkyl, or halogen, R₃ and R₄ may be hydrogen or halogen, X may be O or -CH(-Rx)-, Rx may be hydrogen or C₁₋₄ alkyl, A may be quinoline, quinazoline, pyridine, pyrimidine, thienopyridine, pyrrolopyridine, pyrazolopyridine, imidazopyridine, pyrrolopyrimidine, dihydropyrrolopyrimidine, furopyridine, pyrazolopyrimidine, purine or indazole, and R₅ and R₆ may be each independently hydrogen, amino, halogen, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, di-C₁₋₆ alkylcarbonylamino, C₁₋₆ alkylcarbonylamino, C₁₋₆ alkylamino, C₁₋₆ alkyl-amino-C₁₋₆ alkoxy, or 5- to 9-membered heteroaryl, wherein R₅ and R₆ may be each independently unsubstituted or substituted with C₁₋₆ alkyl; C₁₋₆ alkyl or C₁₋₆ alkyl-amino-C₁₋₆ alkyl substituted with any one of C₁₋₆ alkoxy-C₁₋₆ alkyl-amino, 3- to 9-membered cycloalkyl and 3- to 9-membered heterocycloalkyl; 3- to 9-membered cycloalkyl; or 3- to 9-membered heterocycloalkyl, wherein the cycloalkyl or heterocycloalkyl may have or may not have at least one substituent selected from the group consisting of halogen, oxo, cyano, hydroxy, hydroxy-C₁₋₆ alkyl, amino, di-C₁₋₆ alkylamino, C₁₋₆ alkyl, C₁₋₆ alkoxy, and C₁₋₆ alkoxy-C₁₋₆ alkyl, and the heteroaryl and the heterocycloalkyl may each independently contain at least one heteroatom selected from the group consisting of N, O and S.

According to another embodiment, in Formula 2, R₅ and R₆ may be each independently hydrogen, nitro, amino, halogen, hydroxy, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, di-C₁₋₆ alkylaminocarbonyl, C₁₋₆ alkylcarbonylamino, C₁₋₆ alkylamino, C₁₋₆ alkyl-amino-C₁₋₆ alkoxy, C₆₋₁₀ aryl, C₆₋₁₀ aryl-C₁₋₄ alkyl, or 5- to 9-membered heteroaryl, wherein the amino, the alkyl, the alkoxy, the aryl and the heteroaryl may be each independently unsubstituted or substituted with C₁₋₆ alkyl; C₁₋₆ alkyl or C₁₋₆ alkylamino-C₁₋₆ alkyl substituted with any one of C₁₋₆ alkoxy-C₁₋₆ alkylamino, 3- to 9-membered cycloalkyl and 3- to 9-membered heterocycloalkyl; 3- to 9-membered cycloalkyl; or 3- to 9-membered heterocycloalkyl, and the cycloalkyl or heterocycloalkyl may have or may not have at least one substituent selected from the group consisting of halogen, oxo, cyano, hydroxy, hydroxy-C₁₋₆ alkyl, amino, di-C₁₋₆ alkylamino, C₁₋₆ alkyl, C₁₋₆ alkoxy, and C₁₋₆ alkoxy-C₁₋₆ alkyl, and the heterocyclic ring, heteroaryl and heterocycloalkyl may each independently contain at least one heteroatom selected from the group consisting of N, O and S.

According to another embodiment, in Formula 2, R₅ and R₆ may not be C₆₋₁₀ aryl, C₆₋₁₀ aryl-C₁₋₄ alkyl, or 5- to 9-membered heteroaryl at the same time. According to another embodiment, in Formula 1, R₅ and R₆ may not be substituted with 3- to 9-membered cycloalkyl and 3- to 9-membered heterocycloalkyl substituted with any one of C₁₋₆ alkyl or C₁₋₆ alkylamino-C₁₋₆ alkyl; 3- to 9-membered cycloalkyl; or 3- to 9-membered heterocycloalkyl at the same time. As a specific example, when R₅ includes a ring such as aryl or heteroaryl, R₆ may not include the ring at the same time. In addition, when R₅ is substituted with a group including a ring such as cycloalkyl or heterocycloalkyl, R₆ may not be substituted with a group including the ring at the same time.

As a more specific example, R₅ may be C₆₋₁₀ aryl, C₆₋₁₀ aryl-C₁₋₄ alkyl, or 5- to 9-membered heteroaryl, and R₆ may be hydrogen, nitro, amino, halogen, hydroxy, cyano, C₁₋₆ alkyl, C₁₋₆ haloalkyl, C₁₋₆ alkoxy, amino-C₁₋₆ alkoxy, aminocarbonyl, C₁₋₆ alkylaminocarbonyl, di-C₁₋₆ alkylaminocarbonyl, C₁₋₆ alkylcarbonylamino, C₁₋₆ alkylamino, or C₁₋₆ alkyl-amino-C₁₋₆ alkoxy, wherein R₅ may be unsubstituted or substituted with C₁₋₆ alkyl; or C₁₋₆ alkyl or C₁₋₆ alkylamino-C₁₋₆ alkyl substituted with any one of C₁₋₆ alkoxy-C₁₋₆ alkylamino, 3- to 9-membered cycloalkyl and 3- to 9-membered heterocycloalkyl. In addition, R₆ may be unsubstituted or substituted with 3- to 9-membered cycloalkyl; or 3- to 9-membered heterocycloalkyl, wherein the cycloalkyl or heterocycloalkyl may have or may not have at least one substituent selected from the group consisting of halogen, oxo, cyano, hydroxy, hydroxy-C₁₋₆ alkyl, amino, di-C₁₋₆ alkylamino, C₁₋₆ alkyl, C₁₋₆ alkoxy, and C₁₋₆ alkoxy-C₁₋₆ alkyl, and the heteroaryl and the heterocycloalkyl may each independently contain at least one heteroatom selected from the group consisting of N, O and S.

According to another embodiment, in Formula 2, R₅ and R₆ may be each independently hydrogen, amino, halogen, hydroxy, C₁₋₆ alkoxy, aminocarbonyl, C₁₋₆ alkylaminocarbonyl, di-C₁₋₆ alkylaminocarbonyl, C₁₋₆ alkylcarbonylamino, cyano, C₁₋₄ haloalkyl, C₁₋₆ alkyl, 5- to 9-membered heteroaryl, Ax-(CH₂)ₐ-L1-(CH₂)_{b}-L2-, or Ax-(CH₂)ₐ-L1-(CH₂)_{b}-L2-pyridinyl, wherein Ax may be C₃₋₆ cycloalkyl or 3- to 6-membered heterocycloalkyl, L1 and L2 may be each independently a single bond, -O-, -NH-, -C(=O)-NH-, or -NH-C(=O)-, and a and b may be each independently an integer of 0 to 3, provided that when b is 0, L2 is a single bond, and the cycloalkyl, heteroaryl and heterocycloalkyl may each independently have or may not have one or two substituents selected from the group consisting of halogen, oxo, cyano, hydroxy, hydroxymethyl, C₁₋₆ alkyl, methoxy, methoxymethyl, dimethylamino, and methoxyethylaminomethyl, and the heteroaryl and the heterocycloalkyl may each independently contain at least one heteroatom selected from the group consisting of N, O and S, and the heteroaryl and the heterocycloalkyl may each independently contain at least one heteroatom selected from the group consisting of N, O and S.

Specific examples of the compound represented by Formula 2 are as follows:
40) N-{4-[(6,7-dimethoxyquinolin-4-yl)oxy]-3-fluorophenyl}-5-(4-fluorophenyl)-6-oxo-2,3,5,6-tetrahydrofuro[3,2-*c*]pyridine-7-carboxamide;
41) N-{4-[(6,7-dimethoxyquinolin-4-yl)oxy]-3-fluorophenyl}-6-oxo-5-phenyl-2,3,5,6-tetrahydrofuro[3,2-*c*]pyridine-7-carboxamide;
42) N-{4-[(6,7-dimethoxyquinolin-4-yl)oxy]-3-fluorophenyl}-6-oxo-5-[4-(trifluoromethyl)phenyl]-2,3,5,6-tetrahydrofuro[3,2-*c*]pyridine-7-carboxamide;
43) N-{4-[(6,7-dimethoxyquinolin-4-yl)oxy]-3-fluorophenyl}-6-oxo-5-(3-fluorophenyl)-2,3,5,6-tetrahydrofuro[3,2-*c*]pyridine-7-carboxamide;
44) N-{3-fluoro-4-[(6-methoxyquinolin-4-yl)oxy]phenyl}-5-(4-fluorophenyl)-6-oxo-2,3,5,6-tetrahydrofuro[3,2-*c*]pyridine-7-carboxamide;
45) N-{3-fluoro-4-[(7-methoxyquinolin-4-yl)oxy]phenyl}-5-(4-fluorophenyl)-6-oxo-2,3,5,6-tetrahydrofuro[3,2-c]pyridine-7-carboxamide;
46) N-{4-[(6,7-dimethoxyquinazolin-4-yl)oxy]-3-fluorophenyl}-5-(4-fluorophenyl)-6-oxo-2,3,5,6-tetrahydrofuro[3,2-*c*]pyridine-7-carboxamide;
47) N-{4-[(6-carbamoyl-7-methoxyquinolin-4-yl)oxy]-3-fluorophenyl}-5-(4-fluorophenyl)-6-oxo-2,3,5,6-tetrahydrofuro[3,2-*c*]pyridine-7-carboxamide;
48) N-(3-fluoro-4-{ [7-methoxy-6-(methylcarbamoyl)quinolin-4-yl]oxy}phenyl)-5-(4-fluorophenyl)-6-oxo-2,3,5,6-tetrahydrofuro[3,2-*c*]pyridine-7-carboxamide;
49) N-(4-{ [6-(dimethylcarbamoyl)-7-methoxyquinolin-4-yl]oxy}-3-fluorophenyl)-5-(4-fluorophenyl)-6-oxo-2,3,5,6-tetrahydrofuro[3,2-*c*]pyridine-7-carboxamide;
50) N-[3-fluoro-4-({7-methoxy-6-[(2-morpholinoethyl)carbamoyl]quinolin-4-yl } oxy)phenyl]-5-(4-fluorophenyl)-6-oxo-2,3, 5, 6-tetrahydrofuro[3,2-*c*]pyridine-7-carboxamide;
51) N-(4-{ [6-(ethylcarbamoyl)-7-methoxyquinolin-4-yl]oxy}-3-fluorophenyl)-5-(4-fluorophenyl)-6-oxo-2,3,5,6-tetrahydrofuro[3,2-*c*]pyridine-7-carboxamide;
52) N-{4-[(6-acetamido-7-methoxyquinolin-4-yl)oxy]-3-fluorophenyl}-5-(4-fluorophenyl)-6-oxo-2,3,5,6-tetrahydrofuro[3,2-*c*]pyridine-7-carboxamide;
53) N-(3-fluoro-4-{ [7-methoxy-6-(2-morpholinoacetamido)quinolin-4-yl]oxy}phenyl)-5-(4-fluorophenyl)-6-oxo-2,3,5,6-tetrahydrofuro[3,2-*c*]pyridine-7-carboxamide;
76) N-(3-fluoro-4-{ [6-methoxy-7-(3-morpholinopropoxy)quinolin-4-yl]oxy}phenyl)-5-(4-fluorophenyl)-6-oxo-2,3,5,6-tetrahydrofuro[3,2-*c*]pyridine-7-carboxamide;
77) N-(3-fluoro-4-{ [6-methoxy-7-(3-morpholinopropoxy)quinolin-4-yl]oxy}phenyl)-2-(4-fluorophenyl)-3-oxo-3,5,6,7-tetrahydro-2H-cyclopenta[c]pyridine-4-carboxamide;
78) N-(3-fluoro-4-{[6-methoxy-7-(2-morpholinoethoxy)quinolin-4-yl]oxy}phenyl)-5-(4-fluorophenyl)-6-oxo-2,3,5,6-tetrahydrofuro[3,2-*c*]pyridine-7-carboxamide;
79) N-(3-fluoro-4-{ [6-methoxy-7-(2-morpholinoethoxy)quinolin-4-yl]oxy}phenyl)-6-oxo-5-phenyl-2,3,5,6-tetrahydrofuro[3,2-*c*]pyridine-7-carboxamide;
80) N-[3-fluoro-4-({6-methoxy-7-[3-(4-methylpiperazin-1-yl)propoxy]quinolin-4-yl } oxy)phenyl-5-(4-fluorophenyl)-6-oxo-2,3, 5, 6-tetrahydrofuro[3,2-*c*]pyridine-7-carboxamide;
81) N-[3-fluoro-4-({7-[3-(3-hydroxyazetidin-1-yl)propoxy]-6-methoxyquinolin-4-yl } oxy)phenyl]-5-(4-fluorophenyl)-6-oxo-2,3, 5, 6-tetrahydrofuro[3,2-*c*]pyridine-7-carboxamide;
82) N-[3-fluoro-4-({7-[3-(3-hydroxy-3-methylazetidin-1-yl)propoxy]-6-methoxyquinolin-4-yl } oxy)phenyl]-5-(4-fluorophenyl)-6-oxo-2,3, 5, 6-tetrahydrofuro[3,2-*c*]pyridine-7-carboxamide;
83) N-{3-fluoro-4-[(7-{3-[3-(hydroxymethyl)azetidin-1-yl]propoxy}-6-methoxyquinolin-4-yl)oxy]phenyl }-5-(4-fluorophenyl)-6-oxo-2,3, 5, 6-tetrahydrofuro[3,2-*c*]pyridine-7-carboxamide;
84) N-[3-fluoro-4-({6-methoxy-7-[3-(3-methoxyazetidin-1-yl)propoxy]quinolin-4-yl } oxy)phenyl]-5-(4-fluorophenyl)-6-oxo-2,3, 5, 6-tetrahydrofuro[3,2-*c*]pyridine-7-carboxamide;
85) N-[3-fluoro-4-({6-methoxy-7-[3-(3-methoxy-3-methylazetidin-1-yl)propoxy] quinolin-4-yl } oxy)phenyl]-5-(4-fluorophenyl)-6-oxo-2,3, 5, 6-tetrahydrofuro[3,2-c]pyridine-7-carboxamide;
86) N-[3-fluoro-4-({7-[3-(3-fluoroazetidin-1-yl)propoxy]-6-methoxyquinolin-4-yl } oxy)phenyl]-5-(4-fluorophenyl)-6-oxo-2,3, 5, 6-tetrahydrofuro[3,2-c]pyridine-7-carboxamide;
87) N-[4-({7-[3-(3,3-difluoroazetidin-1-yl)propoxy]-6-methoxyquinolin-4-yl}oxy)-3-fluorophenyl]-5-(4-fluorophenyl)-6-oxo-2,3,5,6-tetrahydrofuro[3,2-*c*]pyridine-7-carboxamide;
88) N-[4-({7-[3-(3-cyanoazetidin-1-yl)propoxy]-6-methoxyquinolin-4-yl}oxy)-3-fluorophenyl]-5-(4-fluorophenyl)-6-oxo-2,3,5,6-tetrahydrofuro[3,2-*c*]pyridine-7-carboxamide;
89) N-[3-fluoro-4-({6-methoxy-7-[3-(3-methylazetidin-1-yl)propoxy]quinolin-4-yl } oxy)phenyl]-5-(4-fluorophenyl)-6-oxo-2,3, 5, 6-tetrahydrofuro[3,2-*c*]pyridine-7-carboxamide;
90) N-[3-fluoro-4-({7-[3-(3-hydroxypyrrolidin-1-yl)propoxy]-6-methoxyquinolin-4-yl } oxy)phenyl]-5-(4-fluorophenyl)-6-oxo-2,3, 5, 6-tetrahydrofuro[3,2-*c*]pyridine-7-carboxamide;
91) N-[3-fluoro-4-({7-[3-(3-hydroxy-3-methylpyrrolidin-1-yl)propoxy]-6-methoxyquinolin-4-yl } oxy)phenyl]-5-(4-fluorophenyl)-6-oxo-2,3, 5, 6-tetrahydrofuro[3,2-*c*]pyridine-7-carboxamide;
92) N-[3-fluoro-4-({6-methoxy-7-[3-(3-methoxypyrrolidin-1-yl)propoxy]quinolin-4-yl } oxy)phenyl]-5-(4-fluorophenyl)-6-oxo-2,3, 5, 6-tetrahydrofuro[3,2-*c*]pyridine-7-carboxamide;
93) N-[3-fluoro-4-({6-methoxy-7-[3-(pyrrolidin-1-yl)propoxy]quinolin-4-yl } oxy)phenyl]-5-(4-fluorophenyl)-6-oxo-2,3, 5, 6-tetrahydrofuro[3,2-*c*]pyridine-7-carboxamide;
94) N-[3-fluoro-4-({7-[3-(3-fluoropyrrolidin-1-yl)propoxy]-6-methoxyquinolin-4-yl } oxy)phenyl]-5-(4-fluorophenyl)-6-oxo-2,3, 5, 6-tetrahydrofuro[3,2-*c*]pyridine-7-carboxamide;
95) N-[4-({7-[3-(3,3-difluoropyrrolidin-1-yl)propoxy]-6-methoxyquinolin-4-yl}oxy)-3-fluorophenyl]-5-(4-fluorophenyl)-6-oxo-2,3,5,6-tetrahydrofuro[3,2-*c*]pyridine-7-carboxamide;
96) N-[3-fluoro-4-({7-[3-(4-hydroxypiperidin-1-yl)propoxy]-6-methoxyquinolin-4-yl } oxy)phenyl]-5-(4-fluorophenyl)-6-oxo-2,3, 5, 6-tetrahydrofuro[3,2-*c*]pyridine-7-carboxamide;
97) N-[3-fluoro-4-({7-[3-(3-hydroxypiperidin-1-yl)propoxy]-6-methoxyquinolin-4-yl } oxy)phenyl]-5-(4-fluorophenyl)-6-oxo-2,3, 5, 6-tetrahydrofuro[3,2-*c*]pyridine-7-carboxamide;
98) N-[3-fluoro-4-({7-[3-(4-hydroxy-4-methylpiperidin-1-yl)propoxy]-6-methoxyquinolin-4-yl } oxy)phenyl]-5-(4-fluorophenyl)-6-oxo-2,3, 5, 6-tetrahydrofuro[3,2-*c*]pyridine-7-carboxamide;
99) N-[3-fluoro-4-({6-methoxy-7-[3-(4-methoxypiperidin-1-yl)propoxy]quinolin-4-yl } oxy)phenyl]-5-(4-fluorophenyl)-6-oxo-2,3, 5, 6-tetrahydrofuro[3,2-*c*]pyridine-7-carboxamide;
100) N-[3-fluoro-4-({6-methoxy-7-[3-(3-methoxypiperidin-1-yl)propoxy]quinolin-4-yl } oxy)phenyl]-5-(4-fluorophenyl)-6-oxo-2,3, 5, 6-tetrahydrofuro[3,2-*c*]pyridine-7-carboxamide;
101) N-[3-fluoro-4-({6-methoxy-7-[3-(4-oxopiperidin-1-yl)propoxy]quinolin-4-yl } oxy)phenyl]-5-(4-fluorophenyl)-6-oxo-2,3, 5, 6-tetrahydrofuro[3,2-*c*]pyridine-7-carboxamide;
102) N-[4-({7-[3-(1,1-dioxidothiomorpholino)propoxy]-6-methoxyquinolin-4-yl}oxy)-3-fluorophenyl]-5-(4-fluorophenyl)-6-oxo-2,3,5,6-tetrahydrofuro[3,2-*c*]pyridine-7-carboxamide;
103) N-[3-fluoro-4-({6-methoxy-7-[3-(piperidin-1-yl)propoxy]quinolin-4-yl } oxy)phenyl]-5-(4-fluorophenyl)-6-oxo-2,3, 5, 6-tetrahydrofuro[3,2-*c*]pyridine-7-carboxamide;
104) N-[3-fluoro-4-({7-[3-(4-fluoropiperidin-1-yl)propoxy]-6-methoxyquinolin-4-yl } oxy)phenyl]-5-(4-fluorophenyl)-6-oxo-2,3, 5, 6-tetrahydrofuro[3,2-*c*]pyridine-7-carboxamide;
105) N-[4-({7-[3-(4,4-difluoropiperidin-1-yl)propoxy]-6-methoxyquinolin-4-yl}oxy)-3-fluorophenyl]-5-(4-fluorophenyl)-6-oxo-2,3,5,6-tetrahydrofuro[3,2-*c*]pyridine-7-carboxamide;
106) N-[3-fluoro-4-({6-methoxy-7-[3-(4-methylpiperidin-1-yl)propoxy]quinolin-4-yl } oxy)phenyl]-5-(4-fluorophenyl)-6-oxo-2,3, 5, 6-tetrahydrofuro[3,2-*c*]pyridine-7-carboxamide;
107) N-[4-({7-[3-(4,4-dimethylpiperidin-1-yl)propoxy]-6-methoxyquinolin-4-yl}oxy)-3-fluorophenyl]-5-(4-fluorophenyl)-6-oxo-2,3,5,6-tetrahydrofuro[3,2-*c*]pyridine-7-carboxamide;
108) N-{3-fluoro-4-[(7-{3-[(3-hydroxycyclobutyl)amino]propoxy}-6-methoxyquinolin-4-yl)oxy]phenyl }-5-(4-fluorophenyl)-6-oxo-2,3, 5, 6-tetrahydrofuro[3,2-*c*]pyridine-7-carboxamide;
109) N-{3-fluoro-4-[(6-methoxy-7-{3-[(3-methoxycyclobutyl)amino]propoxy}quinolin-4-yl)oxy]phenyl}-5-(4-fluorophenyl)-6-oxo-2,3,5,6-tetrahydrofuro[3,2-*c*]pyridine-7-carboxamide;
110) N-[3-fluoro-4-({6-methoxy-7-[3-(oxetan-3-ylamino)propoxy]quinolin-4-yl } oxy)phenyl]-5-(4-fluorophenyl)-6-oxo-2,3, 5, 6-tetrahydrofuro[3,2-*c*]pyridine-7-carboxamide;
111) N-{3-fluoro-4-[(6-methoxy-7-{3-[(oxetan-3-ylmethyl)amino]propoxy}quinolin-4-yl)oxy]phenyl}-5-(4-fluorophenyl)-6-oxo-2,3,5,6-tetrahydrofuro[3,2-*c*]pyridine-7-carboxamide;
112) N-{3-fluoro-4-[(7-{3-[(3-hydroxycyclopentyl)amino]propoxy}-6-methoxyquinolin-4-yl)oxy]phenyl }-5-(4-fluorophenyl)-6-oxo-2,3, 5, 6-tetrahydrofuro[3,2-*c*]pyridine-7-carboxamide;
113) N-{3-fluoro-4-[(7-{3-[(3-hydroxycyclohexyl)amino]propoxy}-6-methoxyquinolin-4-yl)oxy]phenyl}-5-(4-fluorophenyl)-6-oxo-2,3,5,6-tetrahydrofuro[3,2-*c*]pyridine-7-carboxamide;
114) N-(3-fluoro-4-{ [7-(3-{ [(3-hydroxycyclohexyl)methyl]amino}propoxy)-6-methoxyquinolin-4-yl] oxy }phenyl)-5-(4-fluorophenyl)-6-oxo-2,3, 5, 6-tetrahydrofuro[3,2-*c*]pyridine-7-carboxamide;
115) N-{3-fluoro-4-[(6-methoxy-7-f3-[(tetrahydro-2H-pyran-4-yl)amino]propoxy}quinolin-4-yl)oxy]phenyl}-5-(4-fluorophenyl)-6-oxo-2,3,5,6-tetrahydrofuro[3,2-*c*]pyridine-7-carboxamide;
116) N-(3-fluoro-4-{[6-methoxy-7-(3-f[(tetrahydro-2H-pyran-4-yl)methyl]amino}propoxy)quinolin-4-yl]oxy}phenyl)-5-(4-fluorophenyl)-6-oxo-2,3,5,6-tetrahydrofuro[3,2-*c*]pyridine-7-carboxamide;
117) N-[3-fluoro-4-({7-[2-(3 -hydroxyazetidin-1-yl)ethoxy]-6-methoxyquinolin-4-yl } oxy)phenyl]-5-(4-fluorophenyl)-6-oxo-2,3, 5, 6-tetrahydrofuro[3,2-*c*]pyridine-7-carboxamide;
118) N-[3-fluoro-4-({7-[2-(3-hydroxy-3-methylazetidin-1-yl)ethoxy]-6-methoxyquinolin-4-yl } oxy)phenyl]-5-(4-fluorophenyl)-6-oxo-2,3, 5, 6-tetrahydrofuro[3,2-c]pyridine-7-carboxamide;
119) N-{3-fluoro-4-[(7-f2-[3-(hydroxymethyl)azetidin-1-yl]ethoxy}-6-methoxyquinolin-4-yl)oxy]phenyl}-5-(4-fluorophenyl)-6-oxo-2,3,5,6-tetrahydrofuro[3,2-*c*]pyridine-7-carboxamide;
120) N-[3-fluoro-4-({6-methoxy-7-[2-(3-methoxyazetidin-1-yl)ethoxy]quinolin-4-yl } oxy)phenyl]-5-(4-fluorophenyl)-6-oxo-2,3, 5, 6-tetrahydrofuro[3,2-*c*]pyridine-7-carboxamide;
121) N-[3-fluoro-4-({6-methoxy-7-[2-(3-methoxy-3-methylazetidin-1-yl)ethoxy] quinolin-4-yl } oxy)phenyl]-5-(4-fluorophenyl)-6-oxo-2,3, 5, 6-tetrahydrofuro[3,2-*c*]pyridine-7-carboxamide;
122) N-{3-fluoro-4-[(6-methoxy-7-{2-[3-(methoxymethyl)azetidin-1-yl]ethoxy}quinolin-4-yl)oxy]phenyl}-5-(4-fluorophenyl)-6-oxo-2,3,5,6-tetrahydrofuro[3,2-c]pyridine-7-carboxamide;
123) N-[3-fluoro-4-({7-[2-(3-fluoroazetidin-1-yl)ethoxy]-6-methoxyquinolin-4-yl } oxy)phenyl]-5-(4-fluorophenyl)-6-oxo-2,3, 5, 6-tetrahydrofuro[3,2-*c*]pyridine-7-carboxamide;
124) N-[4-({7-[2-(3,3-difluoroazetidin-1-yl)ethoxy]-6-methoxyquinolin-4-yl}oxy)-3-fluorophenyl]-5-(4-fluorophenyl)-6-oxo-2,3,5,6-tetrahydrofuro[3,2-*c*]pyridine-7-carboxamide;
125) N-[4-({7-[2-(3-ethynylazetidin-1-yl)ethoxy]-6-methoxyquinolin-4-yl}oxy)-3-fluorophenyl]-5-(4-fluorophenyl)-6-oxo-2,3,5,6-tetrahydrofuro[3,2-*c*]pyridine-7-carboxamide;
126) N-[3-fluoro-4-({6-methoxy-7-[2-(3-methylazetidin-1-yl)ethoxy]quinolin-4-yl } oxy)phenyl]-5-(4-fluorophenyl)-6-oxo-2,3, 5, 6-tetrahydrofuro[3,2-*c*]pyridine-7-carboxamide;
127) N-[4-({7-[2-(3,3-dimethylazetidin-1-yl)ethoxy]-6-methoxyquinolin-4-yl}oxy)-3-fluorophenyl]-5-(4-fluorophenyl)-6-oxo-2,3,5,6-tetrahydrofuro[3,2-*c*]pyridine-7-carboxamide;
128) N-{4-[(7-{2-[3-(dimethylamino)azetidin-1-yl]ethoxy}-6-methoxyquinolin-4-yl)oxy]-3-fluorophenyl}-5-(4-fluorophenyl)-6-oxo-2,3,5,6-tetrahydrofuro[3,2-*c*]pyridine-7-carboxamide;
129) N-[3-fluoro-4-({7-[2-(3-hydroxypyrrolidin-1-yl)ethoxy]-6-methoxyquinolin-4-yl } oxy)phenyl]-5-(4-fluorophenyl)-6-oxo-2,3, 5, 6-tetrahydrofuro[3,2-*c*]pyridine-7-carboxamide;
130) N-[3-fluoro-4-({7-[2-(3-hydroxy-3-methylpyrrolidin-1-yl)ethoxy]-6-methoxyquinolin-4-yl } oxy)phenyl]-5-(4-fluorophenyl)-6-oxo-2,3, 5, 6-tetrahydrofuro[3,2-c]pyridine-7-carboxamide;
131) N-[3-fluoro-4-({6-methoxy-7-[2-(3-methoxypyrrolidin-1-yl)ethoxy]quinolin-4-yl } oxy)phenyl]-5-(4-fluorophenyl)-6-oxo-2,3, 5, 6-tetrahydrofuro[3,2-*c*]pyridine-7-carboxamide;
132) N-[3-fluoro-4-({6-methoxy-7-[2-(pyrrolidin-1-yl)ethoxy]quinolin-4-yl } oxy)phenyl]-5-(4-fluorophenyl)-6-oxo-2,3, 5, 6-tetrahydrofuro[3,2-*c*]pyridine-7-carboxamide;
133) N-[3-fluoro-4-({7-[2-(3-fluoropyrrolidin-1-yl)ethoxy]-6-methoxyquinolin-4-yl } oxy)phenyl]-5-(4-fluorophenyl)-6-oxo-2,3, 5, 6-tetrahydrofuro[3,2-*c*]pyridine-7-carboxamide;
134) N-[4-({7-[2-(3,3-difluoropyrrolidin-1-yl)ethoxy]-6-methoxyquinolin-4-yl}oxy)-3-fluorophenyl]-5-(4-fluorophenyl)-6-oxo-2,3,5,6-tetrahydrofuro[3,2-*c*]pyridine-7-carboxamide;
135) N-[3-fluoro-4-({7-[2-(4-hydroxypiperidin-1-yl)ethoxy]-6-methoxyquinolin-4-yl } oxy)phenyl]-5-(4-fluorophenyl)-6-oxo-2,3, 5, 6-tetrahydrofuro[3,2-*c*]pyridine-7-carboxamide;
136) N-[3-fluoro-4-({7-[2-(3-hydroxypiperidin-1-yl)ethoxy]-6-methoxyquinolin-4-yl } oxy)phenyl]-5-(4-fluorophenyl)-6-oxo-2,3, 5, 6-tetrahydrofuro[3,2-*c*]pyridine-7-carboxamide;
137) N-[3-fluoro-4-({7-[2-(4-hydroxy-4-methylpiperidin-1-yl)ethoxy]-6-methoxyquinolin-4-yl } oxy)phenyl]-5-(4-fluorophenyl)-6-oxo-2,3, 5, 6-tetrahydrofuro[3,2-*c*]pyridine-7-carboxamide;
138) N-[3-fluoro-4-({6-methoxy-7-[2-(4-methoxypiperidin-1-yl)ethoxy]quinolin-4-yl } oxy)phenyl]-5-(4-fluorophenyl)-6-oxo-2,3, 5, 6-tetrahydrofuro[3,2-*c*]pyridine-7-carboxamide;
139) N-[3-fluoro-4-({6-methoxy-7-[2-(3-methoxypiperidin-1-yl)ethoxy]quinolin-4-yl } oxy)phenyl]-5-(4-fluorophenyl)-6-oxo-2,3, 5, 6-tetrahydrofuro[3,2-*c*]pyridine-7-carboxamide;
140) N-[3-fluoro-4-({6-methoxy-7-[2-(4-oxopiperidin-1-yl)ethoxy]quinolin-4-yl } oxy)phenyl]-5-(4-fluorophenyl)-6-oxo-2,3, 5, 6-tetrahydrofuro[3,2-*c*]pyridine-7-carboxamide;
141) N-[3-fluoro-4-({6-methoxy-7-[2-(3-oxopiperidin-1-yl)ethoxy]quinolin-4-yl } oxy)phenyl]-5-(4-fluorophenyl)-6-oxo-2,3, 5, 6-tetrahydrofuro[3,2-*c*]pyridine-7-carboxamide;
142) N-[4-({7-[2-(1,1-dioxidothiomorpholino)ethoxy]-6-methoxyquinolin-4-yl}oxy)-3-fluorophenyl]-5-(4-fluorophenyl)-6-oxo-2,3,5,6-tetrahydrofuro[3,2-*c*]pyridine-7-carboxamide;
143) N-[3-fluoro-4-({6-methoxy-7-[2-(piperidin-1-yl)ethoxy]quinolin-4-yl}oxy)phenyl]-5-(4-fluorophenyl)-6-oxo-2,3,5,6-tetrahydrofuro[3,2-*c*]pyridine-7-carboxamide;
144) N-[3-fluoro-4-({7-[2-(4-fluoropiperidin-1-yl)ethoxy]-6-methoxyquinolin-4-yl } oxy)phenyl]-5-(4-fluorophenyl)-6-oxo-2,3, 5, 6-tetrahydrofuro[3,2-*c*]pyridine-7-carboxamide;
145) N-[4-({7-[2-(4,4-difluoropiperidin-1-yl)ethoxy]-6-methoxyquinolin-4-yl}oxy)-3-fluorophenyl]-5-(4-fluorophenyl)-6-oxo-2,3,5,6-tetrahydrofuro[3,2-*c*]pyridine-7-carboxamide;
146) N-[3-fluoro-4-({6-methoxy-7-[2-(4-methylpiperidin-1-yl)ethoxy]quinolin-4-yl } oxy)phenyl]-5-(4-fluorophenyl)-6-oxo-2,3, 5, 6-tetrahydrofuro[3,2-*c*]pyridine-7-carboxamide;
147) N-[4-({7-[2-(4,4-dimethylpiperidin-1-yl)ethoxy]-6-methoxyquinolin-4-yl}oxy)-3-fluorophenyl]-5-(4-fluorophenyl)-6-oxo-2,3,5,6-tetrahydrofuro[3,2-*c*]pyridine-7-carboxamide;
148) N-{3-fluoro-4-[(7-{2-[(3-hydroxycyclobutyl)amino]ethoxy}-6-methoxyquinolin-4-yl)oxy]phenyl}-5-(4-fluorophenyl)-6-oxo-2,3,5,6-tetrahydrofuro[3,2-*c*]pyridine-7-carboxamide;
149) N-{3-fluoro-4-[(6-methoxy-7-{2-[(3-methoxycyclobutyl)amino]ethoxy}quinolin-4-yl)oxy]phenyl}-5-(4-fluorophenyl)-6-oxo-2,3,5,6-tetrahydrofuro[3,2-*c*]pyridine-7-carboxamide;
150) N-[3-fluoro-4-({6-methoxy-7-[2-(oxetan-3-ylamino)ethoxy]quinolin-4-yl } oxy)phenyl]-5-(4-fluorophenyl)-6-oxo-2,3, 5, 6-tetrahydrofuro[3,2-*c*]pyridine-7-carboxamide;
151) N-{3-fluoro-4[(6-methoxy-7-{2-[(oxetan-3-ylmethyl)amino]ethoxy}quinolin-4-yl)oxy]phenyl}-5-(4-fluorophenyl)-6-oxo-2,3,5,6-tetrahydrofuro[3,2-*c*]pyridine-7-carboxamide;
152) N-{3-fluoro-4-[(7-{2-[(4-hydroxycyclohexyl)amino]ethoxy}-6-methoxyquinolin-4-yl)oxy]phenyl}-5-(4-fluorophenyl)-6-oxo-2,3,5,6-[3,2-*c*]pyridine-7-carboxamide;
153) N-{3-fluoro-4-[(7-{2-[(3-hydroxycyclohexyl)amino]ethoxy}-6-methoxyquinolin-4-yl)oxy]phenyl}-5-(4-fluorophenyl)-6-oxo-2,3,5,6-tetrahydrofuro[3,2-*c*]pyridine-7-carboxamide;
154) N-(3-fluoro-4-{[7-(2-{[(3-hydroxycyclohexyl)methyl]amino}ethoxy)-6-methoxyquinolin-4-yl]oxy}phenyl)-5-(4-fluorophenyl)-6-oxo-2,3,5,6-tetrahydrofuro[3,2-c]pyridine-7-carboxamide;
155) N-{3-fluoro-4-((6-methoxy-7-{2-[(tetrahydro-2H-pyran-4-yl)amino]ethoxy}quinolin-4-yl)oxy)phenyl}-5-(4-fluorophenyl)-6-oxo-2,3,5,6-tetrahydrofuro[3,2-*c*]pyridine-7-carboxamide;
156) N-{3-fluoro-4-[(6-methoxy-7-{2-[(4-methoxycyclohexyl)amino]ethoxy}quinolin-4-yl)oxy]phenyl}-5-(4-fluorophenyl)-6-oxo-2,3,5,6-tetrahydrofuro[3,2-*c*]pyridine-7-carboxamide;
157) N-(3-fluoro-4-{[7-(2-morpholinoethoxy)quinolin-4-yl]oxy}phenyl)-5-(4-fluorophenyl)-6-oxo-2,3,5,6-tetrahydrofuro[3,2-*c*]pyridine-7-carboxamide;
158) N-(3-fluoro-4-{[7-(3-morpholinopropoxy)quinolin-4-yl]oxy}phenyl)-5-(4-fluorophenyl)-6-oxo-2,3,5,6-tetrahydrofuro[3,2-*c*]pyridine-7-carboxamide;
159) N-(3-fluoro-4-{[7-(3-morpholinopropoxy)quinolin-4-yl]oxy}phenyl)-2-(4-fluorophenyl)-3-oxo-3,5,6,7-tetrahydro-2H-cyclopenta[*c*]pyridine-4-carboxamide;
160) N-[3-fluoro-4-({7-[3-(4-methylpiperazin-1-yl)propoxy]quinolin-4-yl}oxy)phenyl]-5-(4-fluorophenyl)-6-oxo-2,3,5,6-tetrahydrofuro[3,2-*c*]pyridine-7-carboxamide;
161) 7-{2-[3-fluoro-4-({7-[3-(piperidin-1-yl)propoxy]quinolin-4-yl}oxy)phenyl]acetyl}-5-(4-fluorophenyl)-3,5-dihydrofuro[3,2- c]pyridin-6(2H)-one;
162) 7-{2-[3-fluoro-4-({7-[3-(4-methylpiperidin-1-yl)propoxy]quinolin-4-yl}oxy)phenyl]acetyl}-5-(4-fluorophenyl)-3,5-dihydrofuro[3,2-*c*]pyridin-6(2H)-one;
163) N-(3-fluoro-4- { [6-(methylcarbamoyl)-7-(2-morpholinoethoxy)quinolin-4-yl] oxy }phenyl)-5-(4-fluorophenyl)-6-oxo-2,3, 5, 6-tetrahydrofuro[3,2-*c*]pyridine-7-carboxamide;
164) N-(3-fluoro-4- { [-(methylcarbamoyl)-7-(3-morpholinopropoxy)quinolin-4-yl] oxy }phenyl)-5-(4-fluorophenyl)-6-oxo-2,3, 5, 6-tetrahydrofuro[3,2-c]pyridine-7-carboxamide;
165) N-(3-fluoro-4-{[6-methoxy-7-(methylcarbamoyl)quinolin-4-yl]oxy}phenyl)-5-(4-fluorophenyl)-6-oxo-2,3,5,6-tetrahydrofuro[3,2-c]pyridine-7-carboxamide;
166) N-(3-fluoro-4-{[7-(methylcarbamoyl)-6-(2-morpholinoethoxy)quinolin-4-yl] oxy }phenyl)-5-(4-fluorophenyl)-6-oxo-2,3, 5, 6-tetrahydrofuro[3,2-c]pyridine-7-carboxamide;
167) N-(3-fluoro-4-{[6-fluoro-7-(2-morpholinoethoxy)quinolin-4-yl]oxy}phenyl)-5-(4-fluorophenyl)-6-oxo-2,3,5,6-tetrahydrofuro[3,2-c]pyridine-7-carboxamide;
168) N-(3-fluoro-4-{[6-fluoro-7-(3-morpholinopropoxy)quinolin-4-yl]oxy}phenyl)-5-(4-fluorophenyl)-6-oxo-2,3,5,6-tetrahydrofuro[3,2-*c*]pyridine-7-carboxamide;
169) N-(3-fluoro-4-{[7-(2-morpholinoethoxy)-6-(trifluoromethyl)quinolin-4-yl]oxy}phenyl)-5-(4-fluorophenyl)-6-oxo-2,3,5,6-tetrahydrofuro[3,2-*c*]pyridine-7-carboxamide;
170) N-(3-fluoro-4-{[7-(3-morpholinopropoxy)-6-(trifluoromethyl)quinolin-4-yl] oxy }phenyl)-5-(4-fluorophenyl)-6-oxo-2,3, 5, 6-tetrahydrofuro[3,2-*c*]pyridine-7-carboxamide;
171) N-(4-{ [6-chloro-7-(2-morpholinoethoxy)quinolin-4-yl]oxy}-3-fluorophenyl)-5-(4-fluorophenyl)-6-oxo-2,3,5,6-tetrahydrofuro[3,2-*c*]pyridine-7-carboxamide;
172) N-(4-{[6-chloro-7-(3-morpholinopropoxy)quinolin-4-yl]oxy}-3-fluorophenyl)-5-(4-fluorophenyl)-6-oxo-2,3,5,6-tetrahydrofuro[3,2-*c*]pyridine-7-carboxamide;
173) N-(4-{ [6-cyano-7-(2-morpholinoethoxy)quinolin-4-yl]oxy}-3-fluorophenyl)-5-(4-fluorophenyl)-6-oxo-2,3,5,6-tetrahydrofuro[3,2-*c*]pyridine-7-carboxamide;
174) N-(4-{ [6-cyano-7-(3-morpholinopropoxy)quinolin-4-yl]oxy}-3-fluorophenyl)-5-(4-fluorophenyl)-6-oxo-2,3,5,6-tetrahydrofuro[3,2-*c*]pyridine-7-carboxamide;
175) N-(3-fluoro-4-{ [7-methoxy-6-(2-morpholinoethoxy)quinolin-4-yl]oxy}phenyl)-5-(4-fluorophenyl)-6-oxo-2,3,5,6-tetrahydrofuro[3,2-*c*]pyridine-7-carboxamide;
176) N-(3-fluoro-4-{ [7-methoxy-6-(3-morpholinopropoxy)quinolin-4-yl]oxy}phenyl)-5-(4-fluorophenyl)-6-oxo-2,3,5,6-tetrahydrofuro[3,2-*c*]pyridine-7-carboxamide;
177) N-(3-fluoro-4-{[6-(2-morpholinoethoxy)quinolin-4-yl]oxy}phenyl)-5-(4-fluorophenyl)-6-oxo-2,3,5,6-tetrahydrofuro[3,2-*c*]pyridine-7-carboxamide;
178) N-(3-fluoro-4-{[6-(3-morpholinopropoxy)quinolin-4-yl]oxy}phenyl)-5-(4-fluorophenyl)-6-oxo-2,3,5,6-tetrahydrofuro[3,2-*c*]pyridine-7-carboxamide;
179) N-{4-[(6-amino-7-methoxyquinolin-4-yl)oxy]-3-fluorophenyl}-5-(4-fluorophenyl)-6-oxo-2,3, 5, 6-tetrahydrofuro[3,2-*c*]pyridine-7-carboxamide;
180) N-(3-fluoro-4-{[7-methoxy-6-(3-morpholinopropanamido)quinolin-4-yl] oxy }phenyl)-5-(4-fluorophenyl)-6-oxo-2,3, 5, 6-tetrahydrofuro[3,2-*c*]pyridine-7-carboxamide;
181) N-{4-[(7-amino-6-methoxyquinolin-4-yl)oxy]-3-fluorophenyl}-5-(4-fluorophenyl)-6-oxo-2,3,5,6-tetrahydrofuro[3,2-*c*]pyridine-7-carboxamide;
182) N-(3-fluoro-4-{[6-methoxy-7-(2-morpholinoacetamido)quinolin-4-yl]oxy}phenyl)-5-(4-fluorophenyl)-6-oxo-2,3,5,6-tetrahydrofuro[3,2-*c*]pyridine-7-carboxamide;
183) N-(3-fluoro-4-{[6-methoxy-7-(3-morpholinopropanamido)quinolin-4-yl]oxy}phenyl)-5-(4-fluorophenyl)-6-oxo-2,3,5,6-tetrahydrofuro[3,2-*c*]pyridine-7-carboxamide;
184) N-{4-[(7-acetamido-6-methoxyquinolin-4-yl)oxy]-3-fluorophenyl}-5-(4-fluorophenyl)-6-oxo-2,3,5,6-tetrahydrofuro[3,2-*c*]pyridine-7-carboxamide;
185) N-[3-fluoro-4-({7-methoxy-6-[(2-morpholinoethyl)amino]quinolin-4-yl}oxy)phenyl]-5-(4-fluorophenyl)-6-oxo-2,3,5,6-tetrahydrofuro[3,2-*c*]pyridine-7-carboxamide;
186) N-[3-fluoro-4-({7-methoxy-6-[(3-morpholinopropyl)amino]quinolin-4-yl}oxy)phenyl]-5-(4-fluorophenyl)-6-oxo-2,3,5,6-tetrahydrofuro[3,2-*c*]pyridine-7-carboxamide;
187) N-[3-fluoro-4-({6-methoxy-7-[(2-morpholinoethyl)amino]quinolin-4-yl}oxy)phenyl]-5-(4-fluorophenyl)-6-oxo-2,3,5,6-tetrahydrofuro[3,2-*c*]pyridine-7-carboxamide; and
188) N-[3-fluoro-4-({6-methoxy-7-[(3-morpholinopropyl)amino]quinolin-4-yl}oxy)phenyl]-5-(4-fluorophenyl)-6-oxo-2,3,5,6-tetrahydrofuro[3,2-*c*]pyridine-7-carboxamide.

Preferably, the compound represented by Formula 2 may be
N-(3-fluoro-4-{[6-methoxy-7-(2-morpholinoethoxy)quinolin-4-yl]oxy}phenyl)-5-(4-fluorophenyl)-6-oxo-2,3,5,6-tetrahydrofuro[3,2-*c*]pyridine-7-carboxamide; or
N-[4-({7-[3-(3-cyanoazetidin-1-yl)propoxy]-6-methoxyquinolin-4-yl}oxy)-3-fluorophenyl]-5-(4-fluorophenyl)-6-oxo-2,3,5,6-tetrahydrofuro[3,2-*c*]pyridine-7-carboxamide.

Hereinafter, a method for preparing the compound of Formula 2 will be described. The compound of Formula 2 may be prepared by the methods shown in the following reaction schemes, but is not limited to being prepared by these methods. In particular, one of ordinary skill in the art will fully understand that the compound of Formula 2 of the present invention may be prepared by various methods using techniques well known in the art.

The following reaction scheme shows a method for preparing the compound of Formula 2, step by step, and several compounds of Formula 2 may be prepared by changing the reagents and solvents used in the following preparation steps or by changing the reaction sequence.

According to one embodiment, a compound of Formula 2 may be prepared according to the procedures of Reaction Schemes 1 and 2 below. 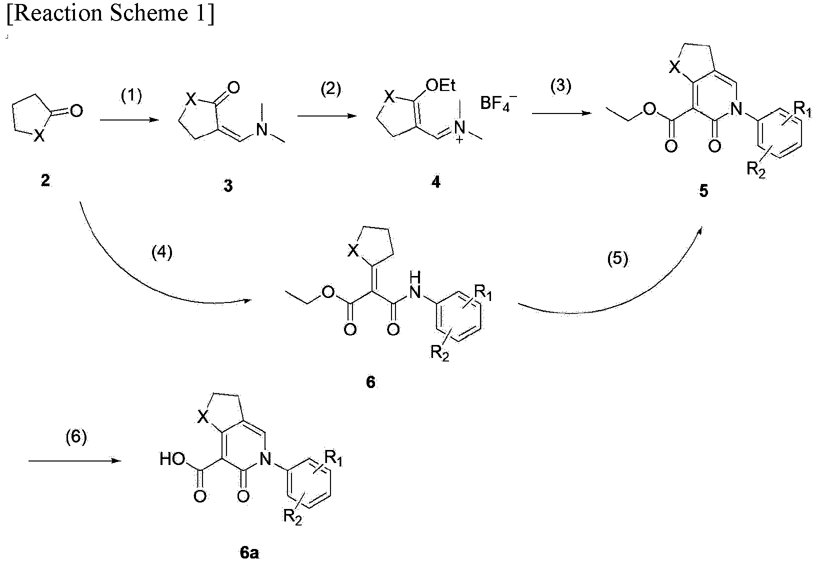 In Reaction Scheme 1, R₁, R₂, and X are as defined in Formula 2 above.

In Reaction Scheme 1, the carboxylic acid compound (6a) is prepared using the lactone-based compound (2), which may be easily obtained commercially or prepared by a known method, as a starting material.

Each step of Reaction Scheme 1 above will be described in detail as follows.

In step 1, the compound of Formula (3) is prepared by formylation reaction of the compound (2), which may be easily obtained commercially, using dimethyl dimethoxy acetal. In general, the reaction may be carried out at a high temperature, but the reaction takes a long time, so the reaction is carried out using a microwave reactor.

In step 2, the compound (4) is prepared using the lactone compound (3) formylated in step 1 above and triethyloxonium tetrafluoroborate, which may be easily obtained commercially. This reaction is carried out under an anhydrous condition, and the reaction is preferably carried out using a solvent that does not adversely affect the reaction, such as N,N-dichloromethane or chloroform. The reaction is generally carried out at room temperature.

In step 3, the compound (4) prepared in step 2 above is reacted with an ethyl-3-amino-3-oxopropionate compound prepared by a conventionally known method in the presence of sodium ethoxide to prepare the cyclized compound (5). This reaction is preferably carried out using an ethanol solvent that does not adversely affect the reaction. The reaction temperature is not particularly limited, but in general, the reaction may be carried out at cold temperature to heating temperature, and the reaction is preferably carried out at room temperature.

Alternatively, in order to prepare the cyclized compound (5), as in step 4, the compound (6) may be prepared by reacting the lactone-based compound (2), which may be easily obtained commercially, as a starting material and an ethyl-3-amino-3-oxopropionate compound prepared by a conventionally known method in the presence of titanium tetrachloride and pyridine. This reaction is preferably carried out using dichloromethane that does not adversely affect the reaction. The reaction temperature is not particularly limited, but in general, the reaction may be carried out at cold temperature to room temperature, and the reaction is preferably carried out initially at cold temperature and carried out at room temperature.

Thereafter, in step 5, the compound (5) is prepared by formylation and cyclization of the compound (6) prepared in step 4 above using dimethyl dimethoxy acetal. In general, the reaction may be carried out at heating temperature and high temperature, but the reaction is preferably carried out at heating temperature.

In step 6, the carboxylic acid compound (6a) is prepared by hydrolyzing the cyclized compound (5) prepared in steps 3 and 5 above. In general, the hydrolysis reaction is carried out using a basic aqueous solution such as sodium hydroxide aqueous solution or lithium hydroxide aqueous solution. This reaction is carried out using a solvent that does not adversely affect the reaction, such as ethanol, methanol, or tetrahydrofuran, in the presence of lithium hydroxide aqueous solution that may be used for the hydrolysis reaction. The reaction temperature is not particularly limited, but in general, the reaction may be carried out at room temperature to heating temperature, and the reaction is preferably carried out at heating temperature to prepare the carboxylic acid compound (6a). 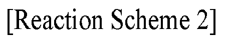 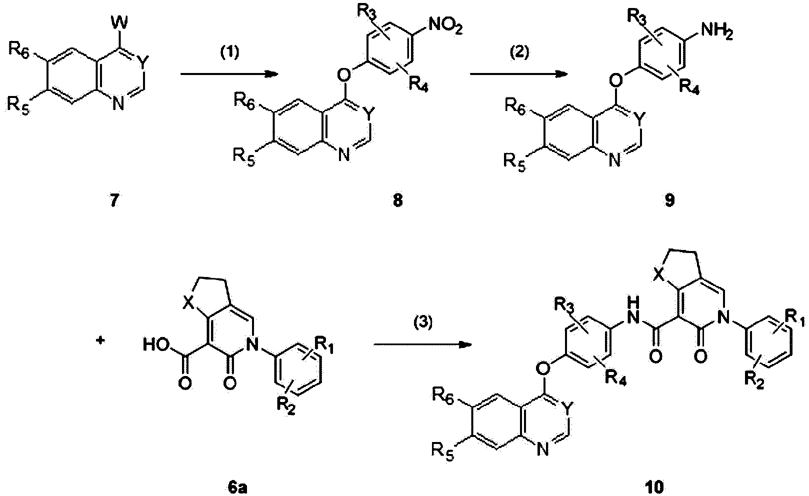 In Reaction Scheme 2, R₁ to R₆, X and Y are as defined in Formula 2 above, and W is a leaving group.

Each step of Reaction Scheme 2 above will be described in detail for preparing the desired compound (10) of the present invention.

In step 1, the monocyclic or bicyclic compound (7), which may be easily obtained commercially or prepared by a known method, is reacted with a nitrophenol compound that may be easily obtained commercially in the presence of a base such as potassium carbonate to prepare the phenoxy compound (8). This reaction is generally an etherification reaction of a phenol compound and is carried out in the presence of a base that may be used in the etherification reaction. Examples of the base that may be used for this purpose include sodium hydride (NaH), potassium carbonate, sodium carbonate, cesium carbonate, sodium or potassium alkoxide, and the like. In addition, the reaction is preferably carried out in the presence of a solvent that does not adversely affect the reaction, and the reaction is carried out using a solvent such as dichloromethane, chloroform, tetrahydrofuran, diethyl ether, toluene, N,N-dimethylformamide, acetonitrile, or diphenyl ether. The reaction temperature is not particularly limited, but in general, the reaction may be carried out at room temperature to heating temperature, and the reaction is preferably carried out at heating temperature.

In step 2, the nitrophenol compound (8) prepared in step 1 above is reduced in the presence of iron and ammonium chloride to prepare the amine compound (9). This reaction is generally a reduction reaction of a nitro compound to an amine, and the reaction may be carried out using various reducing agents such as hydrogen, iron, tin (± chloride), zinc, and the like. In addition, the reaction is preferably carried out using a solvent that does not adversely affect the reaction, such as dichloromethane, ethyl acetate, methanol, ethanol, tetrahydrofuran, or N,N-dimethylformamide, and the reaction is carried out using water as a cosolvent depending on the reaction. The reaction temperature is not particularly limited, but in general, the reaction may be carried out at room temperature to heating temperature, and the reaction is preferably carried out at heating temperature.

In step 3, the desired compound (10) is prepared through a general amidation reaction in which the amine compound (9) prepared in step 2 above and the carboxylic acid compound (6a) prepared in Reaction Scheme 1 are reacted using a coupling reagent. In general, the reaction is carried out using 1-(3-dimethylaminopropyl)-3-ethylcarbodiimide (EDC), 1,3-dicyclohexyl carboimide (DCC), 1,1-carbonyl diimidazole (CDI), 1-[bis(dimethylamino)methylene]-1H-1,2,3-triazolo[4,5-b]pyridinium 3-oxide hexafluorophosphate (HATU), and the like, which may be easily obtained commercially, as a coupling reagent. This reaction may be carried out without using a base, but the reaction is carried out using a solvent that does not adversely affect the reaction, such as acetonitrile, dimethylformamide, or dichloromethane, in the presence of the general bases that may be used in the amidation reaction, such as 4-dimethylaminopyridine, pyridine, triethylamine, diethylisopropylamine, N-methylmorpholine or dimethylphenylamine. The reaction temperature is not particularly limited, but in general, the reaction may be carried out at room temperature to heating temperature, and the reaction is preferably carried out at room temperature to prepare the desired compound (10).

The desired compounds produced in the above reaction scheme may be separated and purified using a conventional method, for example, column chromatography, recrystallization, or the like.

As used herein, the term "halogen" refers to F, Cl, Br or I, unless otherwise stated.

The term "alkyl," unless otherwise specified, refers to a linear or branched saturated hydrocarbon moiety. For example, "C₁₋₁₀ alkyl" refers to an alkyl having a backbone of 1 to 10 carbons. Specifically, C₁₋₁₀ alkyl may include methyl, ethyl, n-propyl, i-propyl, n-butyl, i-butyl, t-butyl, n-pentyl, i-pentyl, t-pentyl, sec-pentyl, neopentyl, hexyl, heptyl, octyl, nonyl, decyl, and the like.

The term "haloalkyl" refers to an alkyl substituted with at least one halogen. Specifically, haloalkyl may be an alkyl substituted with two or more halogens of the same kind or two or more kinds of halogens.

The term "alkoxy", unless otherwise specified, refers to a group having the formula -O-alkyl, in which the alkyl group as defined above is attached to the parent compound through an oxygen atom. The alkyl portion of the alkoxy group may have from 1 to 20 carbon atoms (i.e., C₁-C₂₀ alkoxy), from 1 to 12 carbon atoms (i.e., C₁-C₁₂ alkoxy), or from 1 to 6 carbon atoms (i.e., C₁-C₆ alkoxy). Examples of suitable alkoxy groups include methoxy (-O-CH₃ or -OMe), ethoxy (-OCH₂CH₃ or -OEt), t-butoxy (-O-C(CH₃)₃ or -O-tBu), and the like.

The term "aryl" refers to an aromatic hydrocarbon radical derived by the removal of one hydrogen atom from a carbon atom constituting a parent aromatic ring system. For example, an aryl group may have from 6 to 20 carbon atoms, from 6 to 14 carbon atoms, or from 6 to 10 carbon atoms.

The term "cycloalkyl" refers to a saturated monocycle or polycycle containing only carbon atoms in the ring. The cycloalkyl may have from 3 to 7 carbon atoms as a monocycle, from 7 to 12 carbon atoms as a bicycle, and up to about 20 carbon atoms as a polycycle.

The term "heteroaryl" refers to an aromatic heterocyclyl having at least one heteroatom in the ring. Non-limiting examples of heteroaryl include pyridinyl, pyrrolyl, oxazolyl, indolyl, isoindolyl, purinyl, furanyl, thienyl, benzofuranyl, benzothiophenyl, carbazolyl, imidazolyl, thiazolyl, isoxazolyl, pyrazolyl, isothiazolyl, quinolyl, isoquinolyl, pyridazyl, pyrimidyl, pyrazyl (which may have at least one substituent in the ring), and the like.

The term "heterocycle" refers to an aromatic or non-aromatic ring having at least one heteroatom, which may be saturated or unsaturated, and may be monocyclic or polycyclic. For example, "4 to 10-membered heterocycle" refers to a heterocycle having a backbone of a total of 4 to 10 atoms including heteroatoms and carbon atoms. Specifically, a 4 to 10-membered heterocycle may include azetidine, diazetidine, pyrrolidine, pyrrole, imidazolidine, imidazole, pyrazolidine, pyrazole, oxazolidine, oxazole, isoxazolidine, isoxazole, thiazolidine, thiazole, isothiazolidine, isothiazole, piperidine, pyridine, piperazine, diazine, morpholine, thiomorpholine, azepane, diazepane, and the like.

The term "heterocycloalkyl" refers to a non-aromatic heterocyclyl having at least one heteroatom in the ring. The heterocycloalkyl may have at least one carbon-carbon double bond or carbon-heteroatom double bond in the ring to the extent that the ring is not aromatic due to the presence of the double bond. Non-limiting examples of heterocycloalkyl include azetidinyl, aziridinyl, pyrrolidinyl, piperidinyl, piperazinyl, homopiperazinyl, morpholino, thiomorpholino, tetrahydrofuranyl, tetrahydrothiofuranyl, tetrahydropyranyl, pyranyl (which may have at least one substituent in the ring), and the like.

The term "heteroatom" refers to an atom other than carbon (C), and specifically, may be a nitrogen (N), oxygen (O) or sulfur (S) atom. The above-mentioned heteroaryl and heterocycloalkyl contain at least one heteroatom, and may contain, for example, 1, 1 to 2, 1 to 3, or 1 to 4 heteroatoms.

The term "substitution" refers to the replacement of a hydrogen atom in a molecular structure with a substituent such that the compound is chemically stable from such substitution without exceeding the valence on the designated atom. For example, "group A is substituted with substituent B" or "group A has substituent B" may means that a hydrogen atom bonded to an atom such as carbon constituting the backbone of group A is replaced with substituent B, and group A and substituent B form a covalent bond. Therefore, it is substantially difficult or impossible for a group that does not have a hydrogen atom capable of leaving to have a substituent. Accordingly, in the present specification, when exemplifying the range of combinations of various groups and substituents, including a group that is difficult to have a substituent, combinations of substituents and groups that are obviously impossible to have a substituent should be interpreted as being excluded from the range.

The pharmaceutical composition of the present invention comprises a pharmaceutically acceptable salt of the compound represented by Formula 1 or Formula 2 as an active ingredient.

The pharmaceutically acceptable salt should have low toxicity to humans and should not adversely affect the biological activity and physicochemical properties of the parent compound.

For example, the pharmaceutically acceptable salt may be an acid addition salt formed by a pharmaceutically acceptable free acid.

The free acid may be an inorganic acid or an organic acid, wherein the inorganic acid may be hydrochloric acid, sulfuric acid, nitric acid, phosphoric acid, perchloric acid, bromic acid, and the like, and the organic acid may be acetic acid, methanesulfonic acid, ethanesulfonic acid, p-toluenesulfonic acid, fumaric acid, maleic acid, malonic acid, phthalic acid, succinic acid, lactic acid, citric acid, gluconic acid, tartaric acid, salicylic acid, malic acid, oxalic acid, benzoic acid, embonic acid, aspartic acid, glutamic acid, and the like.

The acid addition salt may be prepared by a conventional method, for example, by dissolving the compound of Formula 1 or Formula 2 in an excess acid aqueous solution, and precipitating the salt using a water-miscible organic solvent such as methanol, ethanol, acetone or acetonitrile.

In addition, the pharmaceutically acceptable salt may be an alkali metal salt (sodium salt, etc.) or an alkaline earth metal salt (potassium salt, etc.).

The alkali metal salt or alkaline earth metal salt may be obtained, for example, by dissolving the compound of Formula 1 or Formula 2 in an excess alkali metal hydroxide or alkaline earth metal hydroxide solution, filtering the undissolved compound salt, and then evaporating and drying the filtrate.

In addition, the compound of the present invention may have a chiral carbon center and thus may exist in the form of R or S isomer, a racemic compound, an individual enantiomer or mixture, an individual diastereomer or mixture. All such stereoisomers and mixtures thereof may fall within the scope of the present invention.

In addition, the compound of the present invention may include a hydrate and a solvate of the compound of Formula 1 or Formula 2. The hydrate and solvate may be prepared using known methods, and they are preferably nontoxic and water-soluble. In particular, preferably, the hydrate and solvate may be one in which 1 to 5 molecules of water and an alcoholic solvent (particularly, ethanol, etc.) are bound, respectively.

The pharmaceutical composition of the present invention may contain, as an active ingredient, the compound represented by Formula 1 or Formula 2 or a pharmaceutically acceptable salt thereof in an amount of about 0.1% by weight to about 90% by weight, specifically about 0.5% by weight to about 75% by weight, more specifically about 1% by weight to about 50% by weight based on the total weight of the composition.

The pharmaceutical composition of the present invention may include conventional and nontoxic pharmaceutically acceptable additives formulated into a preparation according to a conventional method. For example, the pharmaceutical composition may further include a pharmaceutically acceptable carrier, diluent or excipient.

Examples of additives used in the composition of the present invention may include a sweetening agent, a binder, a solvent, a solubilizing agent, a wetting agent, an emulsifying agent, an isotonic agent, an absorbent, a disintegrant, an antioxidant, a preservative, a lubricant, a glidant, a filler, a flavoring agent, and the like. For example, the additive may include lactose, dextrose, sucrose, mannitol, sorbitol, cellulose, glycine, silica, talc, stearic acid, stearin, magnesium stearate, magnesium aluminosilicate, starch, gelatin, gum tragacanth, alginic acid, sodium alginate, methylcellulose, sodium carboxymethylcellulose, agar, water, ethanol, polyethylene glycol, polyvinylpyrrolidone, sodium chloride, calcium chloride, orange essence, strawberry essence, vanilla flavor, and the like.

The composition of the present invention may be formulated in various preparation forms for oral administration (for example, a tablet, a pill, a powder, a capsule, a syrup or an emulsion) or for parenteral administration (for example, an intramuscular, intravenous or subcutaneous injection).

Preferably, the composition of the present invention may be formulated into a preparation for oral administration. In this case, the additives used may include cellulose, calcium silicate, corn starch, lactose, sucrose, dextrose, calcium phosphate, stearic acid, magnesium stearate, calcium stearate, gelatin, talc, surfactant, suspending agent, emulsifying agent, diluent, and the like. Specifically, examples of the glidant may include colloidal silicon dioxide, magnesium silicate, and the like; examples of the diluent may include microcrystalline cellulose, lactose Fast Flo (registered trademark) lactose anhydrous, lactose monohydrate, silicified MCC HD 90, and the like; examples of the disintegrant may include croscarmellose sodium, crospovidone, and the like; and examples of the lubricant may include magnesium stearate, sodium lauryl sulfate, stearic acid, and the like.

In addition, the liquid preparation for oral administration may be exemplified by a suspension, an emulsion, a syrup, and the like, and various excipients such as a wetting agent, a sweetening agent, a perfuming agent, a preservative, and the like, in addition to water and liquid paraffin, which are commonly used simple diluents, may be included.

In addition, the preparation for parenteral administration includes a sterile aqueous solution, a non-aqueous solution, a suspension, an emulsion, a lyophilized preparation and a suppository. As non-aqueous solvents and suspending agents, propylene glycol, polyethylene glycol, vegetable oils such as olive oil, injectable esters such as ethyl oleate, and the like may be used. As the base of the suppository, witepsol, macrogol, Tween 61, cacao butter, laurin butter, glycerogelatin, and the like may be used. On the other hand, injections may include conventional additives such as a solubilizing agent, an isotonic agent, a suspending agent, an emulsifying agent, a stabilizer, and a preservative.

The term "prevention" refers to any action that inhibits the occurrence of or delays the onset of small cell lung cancer by administration of the pharmaceutical composition. The term "treatment" refers to any action that improves or beneficially changes the symptoms of small cell lung cancer by administration of the pharmaceutical composition.

The compound or composition of the present invention may be administered to a patient in a therapeutically effective amount or in a pharmaceutically effective amount.

Herein, the term "therapeutically effective amount" or "pharmaceutically effective amount" refers to an amount of a compound or composition effective for preventing or treating a target disease, and means an amount that is sufficient to treat the disease at a reasonable benefit/risk ratio applicable to medical treatment and does not cause side effects. The level of the effective amount may be determined depending on factors including the health status of the patient, the type and severity of the disease, the activity of the drug, the sensitivity to the drug, administration method, administration time, the route of administration and excretion rate, treatment duration, the combined or concurrently used drugs and other factors well known in the medical field.

The term "administration" means introducing a predetermined drug to a patient by any appropriate method, and the drug may be administered through any general route as long as the drug may reach a target tissue. The route of administration may include intraperitoneal administration, intravenous administration, intramuscular administration, subcutaneous administration, intradermal administration, oral administration, topical administration, intranasal administration, intrapulmonary administration, intrarectal administration, and the like, but is not limited thereto.

The compound or composition of the present invention may be administered as an individual therapeutic agent or in combination with other therapeutic agents, and may be administered sequentially or simultaneously with conventional therapeutic agents, and may be administered singly or multiple times. Taking all of the above factors into consideration, it is important to administer an amount that may provide the maximum effect by the minimum amount with the minimum side effects or without side effects, which may be easily determined by one of ordinary skill in the art.

Specifically, the effective amount of the compound in the composition of the present invention may vary depending on the age, sex, and body weight of the patient, and in general, may be administered from about 0.1 mg to about 1,000 mg, or from about 5 mg to about 200 mg per kg of body weight daily or every other day or may be divided into 1 to 3 times a day. However, since it may be increased or decreased depending on the route of administration, the severity of the disease, sex, body weight, age, and the like, the scope of the present invention is not limited thereto.

Preferably, the compound or composition of the present invention may be administered for tumor therapy in combination with chemotherapy, radiation therapy, immunotherapy, hormone therapy, bone marrow transplantation, stem cell replacement therapy, other biological therapies, surgical intervention or a combination thereof. For example, the compound or composition of the present invention may be used as an adjuvant therapy in combination with other long-term treatment strategies, or may be used to maintain the patient's condition after tumor regression or chemopreventive therapy in a severe patient.

The pharmaceutical composition of the present invention may further comprise one or more active ingredients, and the additional active ingredients may be anti-proliferative compounds such as, aromatase inhibitors, anti-estrogens, topoisomerase I inhibitors, topoisomerase II inhibitors, microtubule active compounds, alkylating compounds, histone deacetylase inhibitors, compounds inducing cell differentiation processes, cyclooxygenase inhibitors, MMP inhibitors, mTOR inhibitors, anti-neoplastics, anti-metabolites, platinum compounds, compounds targeting/reducing protein or lipid kinase activity, anti-angiogenic compounds, compounds targeting, reducing or inhibiting the activity of protein or lipid phosphatase, gonadorelin agonists, anti-androgens, methionine aminopeptidase inhibitors, bisphosphonates, biological reaction modifiers, anti-proliferative antibodies, heparanase inhibitors, inhibitors of Ras tumorigenic isoforms, telomerase inhibitors, proteasome inhibitors, compounds used in the treatment of hematologic malignancies, compounds targeting, reducing or inhibiting the activity of Flt-3, Hsp90 inhibitors, kinesin spindle protein inhibitors, MEK inhibitors, leucovorin, EDG binders, anti-leukemia compounds, ribonucleotide reductase inhibitors, S-adenosylmethionine decarboxylase inhibitors, hemostatic steroids, corticosteroids, other chemotherapy compounds, or photosensitizing compounds, but are not limited thereto.

The additional active ingredient may be a known anticancer agent. Non-limiting examples of the anticancer agent include DNA alkylating agents such as mechloethamine, chlorambucil, phenylalanine, mustard, cyclophosphamide, ifosfamide, carmustine (BCNU), lomustine (CCNU), streptozotocin, busulfan, thiotepa, cisplatin and carboplatin; anticancer antibiotics such as dactinomycin (actinomycin D), doxorubicin (adriamycin), daunorubicin, idarubicin, mitoxantrone, plicamycin, mitomycin C and bleomycin; and plant alkaloids such as vincristine, vinblastine, paclitaxel, docetaxel, etoposide, teniposide, topotecan and irinotecan, and the like.

In this case, the drug may be a compound represented by Formula 1 or Formula 2 or a pharmaceutically acceptable salt thereof.

In this case, the resistance to the EGFR-targeted therapeutic agent is as described above. In addition, the EGFR-targeted therapeutic agent may be at least one selected from the group consisting of cetuximab, gefitinib, erlotinib, apatinib, icotinib, brigatinib, lapatinib, canertinib, AEE788, XL647, zactima, and panitumumab.

In another aspect of the present invention, there is provided the use of the pharmaceutical composition for the prevention or treatment of small cell lung cancer. In this case, the pharmaceutical composition is as described above.

In another aspect of the present invention, there is provided a method for preventing or treating small cell lung cancer, comprising: detecting a mutation in RON in a biological sample derived from a subject suffering from small cell lung cancer, wherein the RON mutation is RON△155 in which exons 5, 6 and 11 are deleted, RON△160 in which exons 5 and 6 are deleted, or RONΔ165 in which exon 11 is deleted; and administering the pharmaceutical composition according to one aspect of the present invention to a subject in which the mutation in RON is detected.

The RON, mutation in RON, pharmaceutical composition, administration, prevention, and treatment are as described above.

The biological sample refers to a sample obtained from the subject. The biological sample may be tissue, blood, plasma, serum, bone marrow fluid, lymph fluid, saliva, tear fluid, mucosal fluid, amniotic fluid, or a combination thereof.

The subject may be a mammal, such as a human, cow, horse, pig, dog, sheep, goat or cat. The subject may be a patient suffering from a disease associated with the mutation in RON, for example small cell lung cancer, or a subject with a high likelihood of suffering from small cell lung cancer

The method may further comprise administering an anticancer agent to the subject. The anticancer agent may be administered simultaneously, separately, or sequentially with the pharmaceutical composition.

The administration method may be oral or parenteral administration. The administration method may be, for example, oral, transdermal, subcutaneous, rectal, intravenous, intraarterial, intraperitoneal, intramuscular, intrasternal, topical, intranasal, intratracheal, or intradermal route. The composition may be administered systemically or locally, alone or in combination with other pharmaceutically active compounds.

The preferred dosage of the pharmaceutical composition varies depending on the condition and body weight of the patient, the severity of the disease, the drug form, the route and duration of administration, but may be appropriately selected by one of ordinary skill in the art.

The dosage may be, for example, in the range of about 0.001 mg/kg to about 100 mg/kg, about 0.01 mg/kg to about 10 mg/kg, or about 0.1 mg/kg to about 1 mg/kg, based on an adult. The administration may be performed once a day, multiple times a day, or once a week, once every two weeks, once every three weeks, or once every four weeks to once a year.

In another aspect of the present invention, there is provided a method of providing information on an anticancer therapeutic agent, comprising: detecting a mutation in RON in a biological sample derived from a subject suffering from small cell lung cancer, wherein the RON mutation is RONΔ155 in which exons 5, 6 and 11 are deleted, RONΔ160 in which exons 5 and 6 are deleted, or RON△165 in which exon 11 is deleted; and providing information that the pharmaceutical composition according to one aspect of the present invention is suitable for the prevention or treatment of small cell lung cancer to a subject in which the mutation in RON is detected.

As used herein, the term "suitable for anticancer therapy" means available for anticancer therapy, and "providing information that it is suitable for anticancer therapy" means providing information that may determine whether it may be selected as a drug available for anticancer therapy.

The biological sample, RON, mutation in RON, pharmaceutical composition, subject, prevention, and treatment are as described above.

### Mode for Carrying out the Invention

Hereinafter, the present invention will be described in more detail by way of the following examples. However, the following examples are only for illustrating the present invention, and the scope of the present invention is not limited to the following examples.

### Preparation Example 1: 5-(4-fluorophenyl)-6-oxo-2,3,5,6-tetrahydrofuro[3,2-c]pyridine-7-carboxylic acid

### Step 1: Synthesis of 3-[(dimethylamino)methylene]-dihydrofuran-2-(3H)-one

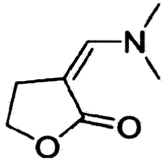

Gamma-butyrolactone (2.69 mL, 35.0 mmol) and dimethyl dimethoxy acetal (11.62 mL, 87.5 mmol) were stirred, and then reacted in a microwave reactor at a temperature of 230°C for at least 70 minutes. The reaction mixture was concentrated to remove excess dimethyl dimethoxy acetal. It was solidified with diethyl ether, and the precipitated solid was filtered while washing with diethyl ether. The filtered solid was concentrated under reduced pressure to obtain the title compound (2.9 g, yield: 59%, a brownish solid).

¹H NMR (500 MHz, CDCl₃) δ 7.13 (s, 1H), 4.24 (t, J = 7.5 Hz, 2H), 3.11 (t, J = 7.5 Hz, 2H), 3.03 (s, 6H); ¹H NMR (500 MHz, DMSO-d₆) δ 7.00 (t, J = 1.5 Hz, 1H), 4.11 (t, J = 7.5 Hz, 2H), 3.06 (t, J = 7.5 Hz, 2H), 3.00 (s, 3H)

### Step 2: Synthesis of 3-[(dimethylamino)methylene]-2-(3H)-dihydrofuranylidene ethyl oxonium tetrafluoroborate

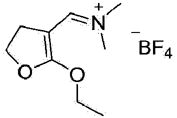

The compound obtained in step 1 (1.085 g, 7.68 mmol) was dissolved in 8 mL of chloroform, and then triethyloxonium tetrafluoroborate (1.729 g, 7.68 mmol) was added thereto, and the mixture was stirred for at least 1 day at room temperature under nitrogen. The reaction mixture was concentrated under reduced pressure, and it was confirmed by nuclear magnetic resonance that the starting material and the product were produced in a ratio of about 15:85, and the next reaction was carried out without purification.

¹H NMR (500 MHz, DMSO-d₆) δ 7.93 (s, 1H), 4.86-4.82 (m, 2H), 4.51 (q, J = 7.0 Hz, 2H), 3.33 (s, 3H), 3.30 (t, J = 8.5 Hz, 2H), 3.26 (s, 3H), 1.36 (t, J = 7.0 Hz, 3H)

### Step 3: Synthesis of ethyl 5-(4-fluorophenyl)-6-oxo-2,3,5,6-tetrahydrofuro[3,2-c]pyridine-7-carboxylate

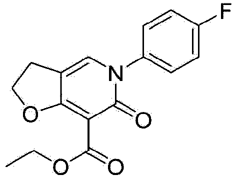

The crude mixture (1.96 g) obtained in step 2 was dissolved in 10 mL of ethanol, and then sodium ethoxide (20 wt% ethanol solution, 2.56 mL, 6.53 mmol) was added in a water bath at 0°C, and then the mixture was slowly stirred for 30 minutes to room temperature. Ethyl 3-{(4-fluorophenyl)amino}-3-oxopropionate (1.47 g, 6.53 mmol) was added to the reaction mixture, and the mixture was stirred at room temperature for at least 20 hours. The reaction mixture was concentrated under reduced pressure and extracted with water and dichloromethane. The separated organic layer was dried over anhydrous magnesium sulfate, filtered, and concentrated under reduced pressure. The resulting residue was purified by column chromatography to obtain the title compound (900 mg, yield: 39% (based on the total yield of steps 2 and 3) /46% (based on the yield of step 3), a yellow solid).

¹H NMR (500 MHz, DMSO-d₆) δ 7.70 (t, J = 1.5 Hz, 1H), 7.42-7.40 (m, 2H), 7.34-7.31 (m, 2H), 4.74 (t, J = 8.0 Hz, 2H), 4.17 (q, J = 7.0 Hz, 2H), 3.05 (td, J = 8.0 Hz, 2H), 1.21 (t, J = 7.0 Hz, 3H)

### Step 4: Synthesis of 5-(4-fluorophenyl)-6-oxo-2,3,5,6-tetrahydrofuro[3,2-c]pyridine-7-carboxylic acid

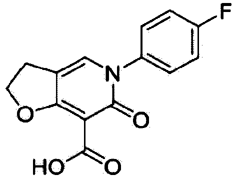

The compound (0.9 g, 2.97 mmol) obtained in step 3 was dissolved in 10 mL of ethanol and 5 mL of distilled water, and then lithium hydroxide monohydrate (249 mg, 5.94 mmol) was added thereto, and the mixture was heated to 50°C and stirred for at least 4 hours. The reaction mixture was concentrated under reduced pressure and extracted with water and dichloromethane. A 1N hydrochloric acid solution was added to the separated aqueous layer, and then extracted with water and dichloromethane. The separated organic layer was dried over anhydrous magnesium sulfate, filtered, and concentrated under reduced pressure. A solid was precipitated in the concentrated residue with a small amount of dichloromethane and diethyl ether, filtered, and then the filtered solid was dried to obtain the title compound (680 mg, yield: 84%, an off-white solid).

¹H NMR (500 MHz, DMSO-d₆) δ 14.5 (bs, OH), 7.97 (s, 1H), 7.54-7.51 (m, 2H), 7.41-7.37 (m, 2H), 4.90 (t, J = 8.5 Hz, 2H), 3.11 (td, J = 8.5, 1.0 Hz, 2H)

### Example 1: 4-ethoxy-N-[3-fluoro-4-({2-[5-(morpholinomethyl)pyridin-2-yl]thieno[3,2-b]pyridin-7-yl}oxy)phenyl]-1-(4-fluorophenyl)-2-oxo-1,2-dihydropyridine-3-carboxamide hydrochloride

The title compound was synthesized by the method described in Example 31 of Korean Patent No. 10-2221689. The title compound was designated as WM-S1-030.

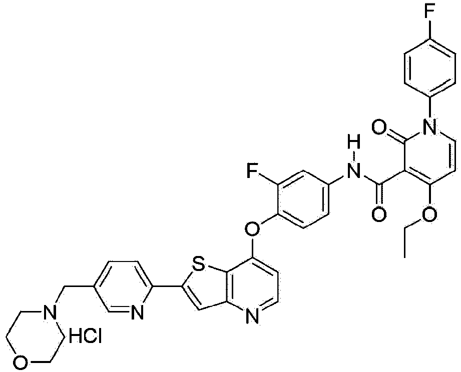

¹H NMR (500MHz, DMSO-d₆) δ 10.66 (s, 1H), 8.81 (s, 1H), 8.60 (d, J = 5.0 Hz, 1H), 8.48 (s, 1H), 8.43 (d, J = 5.0 Hz, 1H), 8.23 (d, J = 5.0 Hz, 1H), 7.96 (d, J = 15.0 Hz, 1H), 7.87 (d, J = 5.0 Hz, 1H), 7.52-7.45 (m, 4H), 7.39-7.36 (m, 2H), 6.83 (d, J = 5.0 Hz, 1H), 6.53 (d, J = 10.0 Hz, 1H), 4.44 (s, 2H), 4.26 (qt, J = 5.0 H, 2H), 3.96-3.94 (m, 2H), 3.77 (t, J = 10.0 Hz, 2H), 3.32-3.30 (m, 2H), 3.14 (qt, J = 10.0 2H), 1.31 (t, J = 5.0 Hz, 3H)

### Example 2: 4-ethoxy-N-(3-fluoro-4-{[2-(5-{[(2-methoxyethyl)amino]methyl}pyridin-2-yl)thieno[3,2-b]pyridin-7-yl]oxy}phenyl)-2-oxo-1-phenyl-1,2-dihydropyridine-3-carboxamide hydrochloride

The title compound was synthesized by the method described in Example 1 of Korean Patent No. 10-2221689.

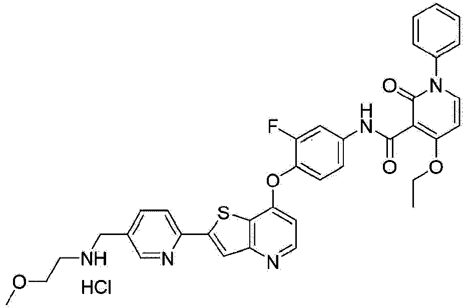

¹H NMR (500 MHz, DMSO-d₆) δ 10.70 (brs, 1H), 9.50 (brs, 2H), 8.80 (s, 1H), 8.69 (d, J = 5.0 Hz, 1H), 8.47 (s, 1H), 8.44 (d, J = 5.0 Hz, 1H), 8.22 (dd, J = 10.0 and 5.0 Hz, 1H), 7.99 (d, J = 10.0 Hz, 1H), 7.88 (d, J = 5.0 Hz, 1H), 7.57-7.40 (m, 7H), 6.97 (d, J = 5.0 Hz, 1H), 6.53 (d, J = 5.0 Hz, 1H), 4.29-4.25 (m, 4H), 3.65 (t, J = 5.0 Hz, 2H), 3.31 (s, 3H), 3.16-3.12 (m, 2H), 1.31 (t, J = 5.0 Hz, 3H)

### Example 3: 4-ethoxy-N-13-fluoro-4-[(2-15-[(-methylpiperazin-1-yl)methyl]pyridin-2-yl}thieno[3,2-b]pyridin-7-yl)oxy]phenyl}-2-oxo-1-phenyl-1,2-dihydropyridine-3-carboxamide hydrochloride

The title compound was synthesized by the method described in Example 34 of Korean Patent No. 10-2221689.

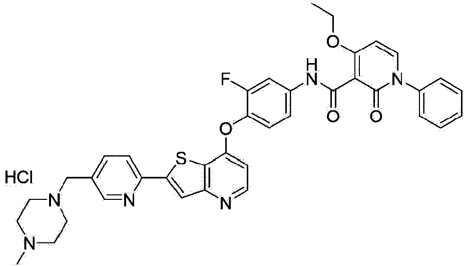

¹H NMR (500MHz, DMSO-d₆) δ 10.68 (s, 1H), 8.82 (brs, 1H), 8.61 (d, J = 5.0 Hz, 1H), 8.47 (s, 1H), 8.41 (d, J = 5.0 Hz, 1H), 8.23 (s, 1H), 7.97 (d, J = 15.0 Hz, 1H), 7.87 (d, J = 5.0 Hz, 1H), 7.56-7.46 (m, 5H), 7.42-7.40 (m, 2H), 6.86 (d, J = 5.0 Hz, 1H), 6.52 (d, J = 5.0 Hz, 1H), 4.26 (qt, J = 5.0 H, 2H), 3.93 (brs, 8H), 3.58 (brs, 2H), 2.81 (s, 3H), 1.31 (t, J = 5.0 Hz, 3H)

### Example 4: N-(3-fluoro-4-{[6-methoxy-7-(2-morpholinoethoxy)quinolin-4-yl]oxy}phenyl)-5-(4-fluorophenyl)-6-oxo-2,3,5,6-tetrahydrofuro[3,2-c]pyridine-7-carboxamide

### Step 1: Synthesis of 4-{2-[(4-chloro-6-methoxyquinolin-7-yl)oxy]ethyl}morpholine

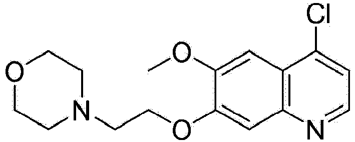

4-chloro-6-methoxyquinolin-7-ol (500 mg, 2.39 mmol) was dissolved in dimethylformamide, and then cesium carbonate (2.33 g, 7.16 mmol), sodium iodide (536 mg, 3.58 mmol), and 4-((2-chloroethyl)morpholine) hydrochloride (666 mg, 3.58 mmol) were sequentially added and stirred at a temperature of 60°C overnight. Thereafter, the mixture was extracted with ethyl acetate and water, dried over anhydrous sodium sulfate, then filtered, and concentrated under reduced pressure. The resulting residue was purified by column chromatography to obtain the title compound (310 mg, yield: 40%, an ivory solid).

¹H NMR (500 MHz, CDCl₃) δ 8.57(d, J = 5.2 Hz, 1H), 7.40(d, J = 6.0 Hz, 2H), 7.35(d, J = 5.2 Hz, 1H), 4.33 (t, J = 6.4Hz, 2H), 4.02(s, 3H), 3.74 (t, J = 4.8Hz, 4H), 2.95 (t, J = 6.0Hz, 2H), 3.74 (t, J = 5.2Hz, 4H) δ 8.58 (d, J = 5.0 Hz, 1H), 7.42 (s, 1H), 7.40 (s, 1H), 7.36 (d, J = 5.0 Hz, 1H), 4.28 (t, J = 5.0 Hz, 2H), 4.03 (s, 3H), 3.85 (m, 4H), 2.65 (m, 4H), 2.32 (m, 2H), 2.15 (m, 2H)

### Step 2: Synthesis of 4-(2-{ [4-(2-fluoro-4-nitrophenoxy)-6-methoxyquinolin-7-yl] oxy } ethyl)morpholine

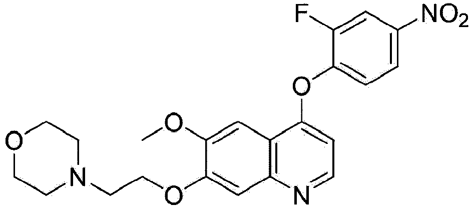

The compound prepared in step 1 (310 mg, 0.96 mmol) was dissolved in diphenyl ether, and then anhydrous potassium carbonate (199 mg, 1.44 mmol) and 2-fluoro-4-nitrophenol (302 mg, 1.92 mmol) were sequentially added and stirred at a temperature of 220°C overnight. Thereafter, the mixture was cooled to room temperature, extracted with ethyl acetate and water, and then dried over anhydrous sodium sulfate, filtered, and concentrated under reduced pressure. The resulting residue was purified by column chromatography to obtain the title compound (362 mg, yield: 85%, a yellow solid).

¹H NMR (500 MHz, DMSO-d₆) δ 8.57(d, J = 5.5 Hz, 1H), 8.47-8.45(dd, J = 3.0 Hz, J = 10.5 Hz, 1H), 8.21(m, 1H), 7.63 (t, J = 8.5Hz, 1H), 7.48(s, 1H), 7.45(s, 1H), 6.78(d, J = 5.0 Hz, 1H), 4.30 (t, J = 5.5Hz, 2H), 3.92(s, 3H), 3.60 (t, J = 4.5Hz, 4H), 2.80 (t, J = 5.5Hz, 2H), 2.52-2.49 (m, 4H, partially overlapped with DMSO)

### Step 3: Synthesis of 3-fluoro-4-{ [6-methoxy-7-(2-morpholinoethoxy)quinolin-4-yl]oxy}aniline

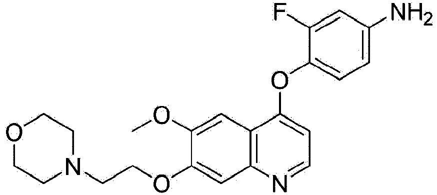

The compound prepared in step 2 (360 mg, 0.81 mmol) was dissolved in ethanol and water, and then iron (136 mg, 2.43 mmol) and ammonium chloride (433 mg, 8.10 mmol) were sequentially added at room temperature, and the mixture was heated to a temperature of 80°C and stirred for 4 hours. After completion of the reaction, it was filtered using a Celite pad and concentrated under reduced pressure. The resulting residue was extracted with dichloromethane and water. The separated organic layer was dried over anhydrous sodium sulfate, concentrated under reduced pressure, and then filtered with hexane to obtain the title compound (280 mg, yield: 83%, a yellow solid).

¹H NMR (500 MHz, DMSO-d₆) δ 8.45 (d, J = 5.0 Hz, 1H), 7.50 (s, 1H), 7.41 (s, 1H), 7.07 (t, J = 10.0 Hz, 1H), 6.54 (d, J = 10.0 Hz, 1H), 6.47 (d, J = 10.0 Hz, 1H), 6.38 (d, J = 5.0 Hz, 1H), 5.50 (brs, 2H), 4.27 (t, J = 10.0 Hz, 2H), 3.94 (s, 3H), 3.60 (t, J = 5.0 Hz, 4H), 2.79 (brs, 2H) 2.52 (brs, 4H, partially overlapped with DMSO)

### Step 4: Synthesis of N-(3-fluoro-4-{[6-methoxy-7-(2-morpholinoethoxy)quinolin-4-yl] oxy }phenyl)-5-(4-fluorophenyl)-6-oxo-2,3, 5, 6-tetrahydrofuro[3,2-c]pyridine-7-carboxamide

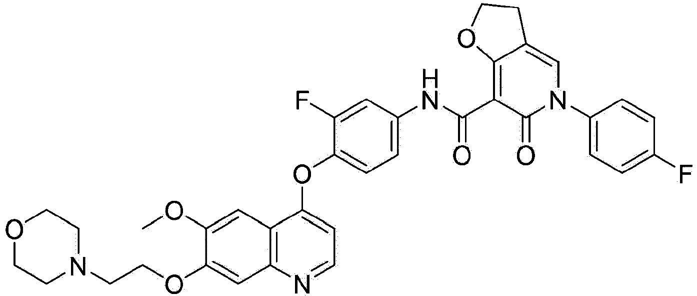

The compound prepared in Preparation Example 1 (149 mg, 0.54 mmol) was dissolved in dimethylformamide, and then 1-[bis(dimethylamino)methylene]-1H-1,2,3-triazolo[4,5-b]pyridinium 3-oxide hexafluorophosphate (HATU, 205 mg, 0.54 mmol), diisopropylethylamine (293 µL, 1.80 mmol), and the compound prepared in step 3 (150 mg, 0.36 mmol) were sequentially added and stirred at room temperature overnight. Thereafter, the mixture was extracted with ethyl acetate and saturated sodium bicarbonate aqueous solution, and then dried over anhydrous sodium sulfate, filtered, and concentrated under reduced pressure. The resulting residue was purified by column chromatography to obtain the title compound (170 mg, yield: 70%, a white solid).

¹H NMR (500 MHz, DMSO-d₆) δ 11.89 (s, 1H), 8.48 (d, J = 5.0 Hz, 1H), 8.00 (d, J = 10.0 Hz, 1H), 7.88 (s, 1H), 7.54-7.49 (m, 3H), 7.45-7.37 (m, 5H), 6.48 (d, J = 5.0 Hz, 1H), 4.85 (t, J = 10.0 Hz, 2H), 4.32 (brs, 2H), 3.95 (s, 3H), 3.63 (brs, 4H), 3.11 (t, J = 10.0 Hz, 2H), 2.52 (m, 2H, partially overlapped with DMSO), 2.50 (m, 4H, overlapped with DMSO)

### Example 5: N-[4-(17-[3-(3-cyanoazetidin-1-yl)propoxy]-6-methoxyquinolin-4-yl}oxy)-3-fluorophenyl]-5-(4-fluorophenyl)-6-oxo-2,3,5,6-tetrahydrofuro[3,2-c]pyridine-7-carboxamide

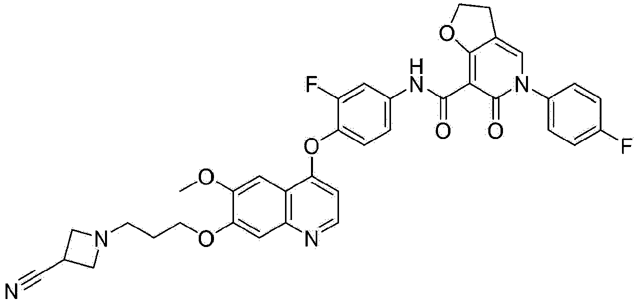

The title compound (yield: 57%, an off-white solid) was obtained in the same manner as in the synthetic route of Example 4 above except that azetidine-3-carbonitrile was used as a starting material.

¹H NMR (400 MHz, CDCl₃) δ 12.04 (S, 1H), 8.48 (d, J = 5.2 Hz, 1H), 8.03 (dd, J = 12.4, 2.4 Hz, 1H), 7.57 (s, 1H), 7.40-7.37 (m, 5H), 7.26-7.22 (m, 2H), 7.17 (t, J = 8.8 Hz, 1H), 6.44 (d, J = 5.6 Hz, 1H), 5.01 (t, J = 8.4 Hz, 2H), 4.26 (t, J = 6.4 Hz, 2H), 4.03 (s, 3H), 3.64 (t, J = 11.6 Hz, 4H), 3.20 (t, J = 8.0 Hz, 2H), 2.81 (t, J = 6.8 Hz, 2H), 2.05 (q, J = 6.8 Hz, 3H)

### Experimental Example 1. Confirmation of cell killing efficacy in small cell lung cancer cell line (H417: mRON△155)

The H417 cell line, which is a small cell lung cancer cell line of a mutation RONΔ155 type, was counted, and 2×10⁵ cells were seeded in a 60 mm dish, respectively, and 24 hours later, treated with 1 µM of each of the compounds synthesized in Example 1 (WM-S1-030), Example 2, Example 3, Example 4, and Example 5 for 72 hours. As a positive control, a known RON inhibitor, BMS-777607 compound (1 µM), was used. BMS-777607 is N-{4-[(2-amino-3-chloro-4-pyridinyl)oxy]-3-fluorophenyl}-4-ethoxy-1-(4-fluorophenyl)-2-oxo-1,2-dihydro-3-pyridinecarboxamide, which is compound 1 disclosed in US Patent No. 8,536,200 B2. BMS-777607 is a well known RON inhibitor.

After 72 hours, all of the medium was transferred to a 15 mL conical tube and washed once with 1 × PBS (phosphate buffered saline), and then the cells were harvested. Thereafter, the supernatant was removed by centrifugation at 1,000 rpm for 3 minutes, and propidium iodide (PI) was diluted in a FACS (Fluorescence-Activated Cell Sorting) buffer (PBS + 2% FBS) at a ratio of 1:1000. The prepared buffer was added adequately depending on the number of cells, and then it was transferred to a FACS tube and reacted for 10 minutes. Thereafter, it was measured using a cytometer (FACS Analyzer).

As a result, it was confirmed that cell death was significantly induced in the cell lines treated with the compounds of Examples 1 (WM-S 1-030) to 5. On the other hand, it was confirmed that the response to the drug was low when treated with the positive control (FIG. 1).

### Experimental Example 2. Confirmation of tumor growth inhibition efficacy by WM-S1-030 in small cell lung cancer cell line (H417: mRON△155) transplanted animal model

It was confirmed whether tumor growth was inhibited by administering WM-S1-030 synthesized in Example 1 above at a dose of 30 mpk to a mouse model transplanted with the H417 cell line, which is a small cell lung cancer cell line of a mutation RONΔ155 type.

Specifically, 5-week-old female BALB/c-nude mice were purchased and acclimatized for 1 week. Thereafter, H417 (5×10⁶ cells/mouse), a small cell lung cancer cell line of a mutation RONΔ155 type, was diluted in PBS (phosphate buffered saline), and 100 µL was injected subcutaneously into the right dorsal side of the mouse. When the size of the tumor reached about 100 mm³, WM-S1-030 was orally administered. At this time, the group orally administered with the vehicle was used as a negative control. The drug was administered once a day for 3 weeks, and the tumor size and body weight were measured twice a week. After the drug administration was completed, the mice were euthanized, the tumor was extracted, and the weight was measured.

As a result, it was confirmed that tumor growth was significantly inhibited in mice administered with WM-S1-030 synthesized in Example 1 above at a dose of 30 mpk (FIG. 2).

### Experimental Example 3. Analysis of protein expression change by WM-S1-030 in small cell lung cancer cell line (H417: mRON△155) transplanted animal model

WM-S1-030 synthesized in Example 1 above was administered at each concentration (3 mpk, 10 mpk, 30 mpk) to a mouse model transplanted with the H417 cell line, which is a small cell lung cancer cell line of a mutation RONΔ155 type, and then the tumor was extracted, and the expression changes of the proteins were analyzed by immunochemical staining.

Specifically, paraffin slides were prepared using tissues isolated from the sacrificed mice. The tissue sections were deparaffinized and rehydrated, immersed in the target recovery buffer 1× (ScyTek Laboratories, Cat# CBB999) and heated for 15 minutes to perform an antigen recovery process. After the reaction, the tissue sections were washed in Tris buffered saline-0.05% Tween 20 (TBS-T)(T&I, BTT-9310) for 5 minutes, and then the reaction was performed with a non-specific antibody reaction blocking reagent (vector, SP-5035) for 1 hour.

The tissue sections were cultured at 4°C overnight with mRON (Abcam, ab52927, 1:50), pTyr-mRON (Mybioscience, MBS462024, 1:100), cleaved caspase-3 (Asp175)(R&D Systems, MAB835, 1:50) and then washed with TBS-T for 5 minutes, and then endogenous peroxidase activity was blocked using a peroxidase blocking reagent (Cellmarque, 925B-05). Thereafter, the tissue sections were washed with TBS-T for 5 minutes and then incubated with a rabbit secondary antibody (vector, PK-6101) at room temperature for 1 hour. The tissue sections were washed with TBS-T for 5 minutes and then colored with diaminobenzidine (cat# SK-4100). In order to stain the nuclei of the tissues, they were stained with hematoxylin (ScyTek Laboratories, HMM999) for 1 minute, dehydrated, and then encapsulated.

As a result, it was confirmed that the expression of pTyr-mRON was decreased in the group administered with WM-S1-030 synthesized in Example 1 above, and the expression of cleaved caspase-3 was induced (FIG. 3).

### Experimental Example 4. Analysis of RON mutation in small cell lung cancer cell line

Seven small cell lung cancer cell lines were harvested, and RNA extraction (Preparation) was carried out using Trizol (Invitrogen). From the obtained RNA, cDNA was synthesized using Bioneer's AccuPower^{®} RT PreMix kit. Using the synthesized cDNA, deletion of exons 5 and 6, and deletion of exon 11 were confirmed by PCR. The primers used are shown in Table 1 below.

**[Table 1]**

| **Primer Name** | **Sequence** | **SEQ ID NO** | **Note** |
|---|---|---|---|
| RON d5_6 sense primer | F: 5'-GAGCTGGTCAGGTCACTAAAC-3' | 5 | Exon 5 & 6 deletion |
| RON d5_6 antisense primer | R: 5'-CAGACACTCAGTCCCATTGAC-3' | 6 | Exon 5 & 6 deletion |
| RON d11 sense primer | F: 5'-ATCTGTGGCCAGCATCTAAC-3' | 7 | Exon 11 deletion |
| RON d11 antisense primer | R: 5'-AAAGGCAGCAGGATACCAAG-3' | 8 | Exon 11 deletion |

The PCR-completed product was confirmed by gel electrophoresis, and the band was extracted using QIAGEN's QIAquick Gel Extraction Kit, and then Macrogen was requested to perform Sanger sequencing, and the sequence was analyzed to confirm whether RON was mutated.

As a result of analyzing a total of 7 small cell lung cancer cell lines of a KRAS wild type, one mRONΔ160 in which exons 5 and 6 are deleted (H889), one mRONΔ165 in which exon 11 is deleted (H187), and two mRONΔ155 in which exons 5, 6 and 11 are all deleted (H417, H146) were identified. In addition, one sample in which mRONΔ155 and the form in which only exon 6 is deleted exist as hetero (HCC33), and two samples in which mRONΔ165 and the form in which only exon 6 is deleted exist as hetero (H69, H719) were identified (FIG. 4).

## Claims

1. A pharmaceutical composition for preventing or treating small cell lung cancer, comprising a compound represented by Formula 1 or Formula 2 below or a pharmaceutically acceptable salt thereof as an active ingredient: 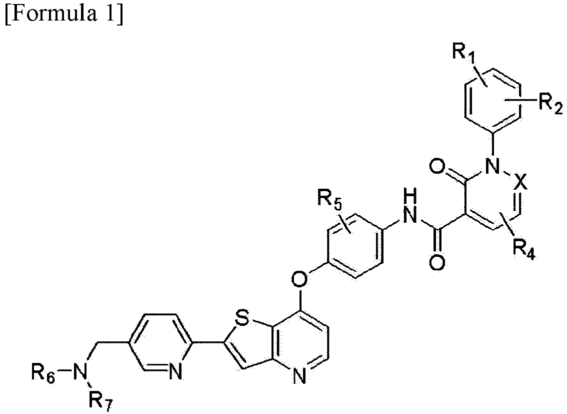 in Formula 1,
R₁ and R₂ are each independently H, halogen, C₁₋₁₀ alkoxy or haloC₁₋₁₀ alkyl;
X is -C(-R₃)= or -N=;
R₃ and R₄ are each independently H, halogen, C₁₋₁₀ alkyl, or C₁₋₁₀ alkoxy;
R₅ is H, halogen, or C₁₋₁₀ alkyl;
R₆ and R₇ are taken together with the N atom to which they are attached to form a 4 to 10-membered heterocycle, or R₆ is -C₂H₄-O-CH₃, and R₇ is H, methyl or t-butoxycarbonyl; and
the heterocycle further has or does not have 1 or 2 heteroatoms selected from the group consisting of N, O and S in addition to the N atom to which R₆ and R₇ are attached, and the heterocycle is also unsubstituted or substituted with at least one selected from halogen and C₁₋₆ alkyl. 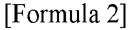 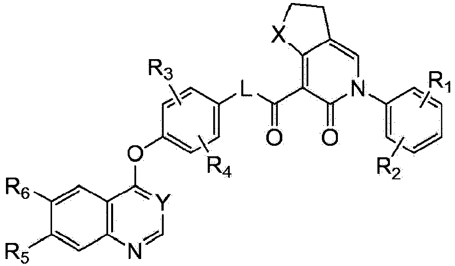 in Formula 2,
L is -NH- or -CH₂-,
R₁ to R₄ are each independently hydrogen, halogen, hydroxy, cyano, C₁₋₄ alkyl, C₁₋₄ alkoxy, C₁₋₄ haloalkyl, C₂₋₄ alkenyl, C₂₋₄ alkynyl, C₃₋₇ cycloalkyl, C₆₋₁₀ aryl, 5- to 9-membered heteroaryl or 3- to 9-membered heterocycloalkyl,
X is O, S, -CH(-Rx)- or -N(-Rx)-,
Rx is hydrogen, C₁₋₄ alkyl, C₁₋₄ alkoxy, C₁₋₄ haloalkyl, C₂₋₄ alkenyl, C₂₋₄ alkynyl, C₆₋₁₀ aryl, C₆₋₁₀ aryl-C₁₋₄ alkyl, or 3- to 9-membered heterocycloalkyl,
Y is -N= or -CH=, and
R₅ and R₆ are each independently hydrogen, amino, halogen, cyano, C₁₋₆ alkyl, C₁₋₆ haloalkyl, C₁₋₆ alkoxy, amino-C₁-₆ alkoxy, aminocarbonyl, C₁₋₆ alkylaminocarbonyl, di-C₁-₆ alkylcarbonylamino, C₁₋₆ alkylcarbonylamino, C₁₋₆ alkylamino, or C₁₋₆ alkyl-amino-C₁-₆ alkoxy,
wherein R₅ and R₆ are each independently unsubstituted or substituted with 3- to 9-membered cycloalkyl; or 3- to 9-membered heterocycloalkyl,
the cycloalkyl or heterocycloalkyl has or does not have at least one substituent selected from the group consisting of halogen, oxo, cyano, hydroxy, hydroxy-C₁-₆ alkyl, amino, di-C₁-₆ alkylamino, C₁₋₆ alkyl, C₁₋₆ alkoxy, and C₁₋₆ alkoxy-C₁-₆ alkyl, and
the heterocycloalkyl contains 1 to 4 heteroatoms selected from the group consisting of N, O and S.

2. The pharmaceutical composition according to claim 1, wherein the compound represented by Formula 1 is a compound selected from the group consisting of:
4-ethoxy-N-[3-fluoro-4-({2-[5-(morpholinomethyl)pyri din-2-yl]thieno[3,2-b]pyri din-7-yl}oxy)phenyl]-1-(4-fluorophenyl)-2-oxo-1,2-dihydropyridine-3-carboxamide;
4-ethoxy-N-(3-fluoro-4-{ [2-(5-{ [(2-methoxy ethyl)amino]methyl}pyri din-2-yl)thieno[3,2-b]pyridin-7-yl]oxy}phenyl)-2-oxo-1-phenyl-1,2-dihydropyridine-3-carboxamide; and
4-ethoxy-N-{3-fluoro-4-[(2-{5-[(-methylpiperazin-1-yl)methyl]pyridin-2-yl}thieno[3,2-b]pyridin-7-yl)oxy]phenyl}-2-oxo-1-phenyl-1,2-dihydropyridine-3-carboxamide.

3. The pharmaceutical composition according to claim 1, wherein the compound represented by Formula 2 is
N-(3-fluoro-4-((6-methoxy-7-(2-morpholinoethoxy)quinolin-4-yl)oxy)phenyl)-5-(4-fluorophenyl)-6-oxo-2,3,5,6-tetrahydrofuro[3,2-c]pyridine-7-carboxamide; or
N-(4-((7-(3-(3-cyanoazetidin-1-yl)propoxy)-6-methoxyquinolin-4-yl)oxy)-3-fluorophenyl)-5-(4-fluorophenyl)-6-oxo-2,3,5,6-tetrahydrofuro[3,2-c]pyridine-7-carboxamide.

4. The pharmaceutical composition for preventing or treating small cell lung cancer according to claim 1, wherein the small cell lung cancer is one in which a RON (Recepteur d'origine nantais) mutation is present.

5. The pharmaceutical composition for preventing or treating small cell lung cancer according to claim 1, wherein the small cell lung cancer has resistance to an EGFR-targeted therapeutic agent.

6. The pharmaceutical composition for preventing or treating small cell lung cancer according to claim 4, wherein the RON mutation is RONΔ155 in which exons 5, 6 and 11 are deleted, RON△160 in which exons 5 and 6 are deleted, or RON△165 in which exon 11 is deleted.

7. The pharmaceutical composition for preventing or treating small cell lung cancer according to claim 5, wherein the EGFR-targeted therapeutic agent is at least one selected from the group consisting of cetuximab, gefitinib, erlotinib, apatinib, icotinib, brigatinib, lapatinib, canertinib, AEE788, XL647, zactima, and panitumumab.

8. A method for preventing or treating small cell lung cancer, comprising:
detecting a mutation in RON in a biological sample derived from a subject suffering from small cell lung cancer,
wherein the RON mutation is RONΔ155 in which exons 5, 6 and 11 are deleted, RON△160 in which exons 5 and 6 are deleted, or RON△165 in which exon 11 is deleted; and
administering the pharmaceutical composition according to claim 1 to a subject in which the mutation in RON is detected.

9. A method of providing information on an anticancer therapeutic agent, comprising:
detecting a mutation in RON in a biological sample derived from a subject suffering from small cell lung cancer,
wherein the RON mutation is RONΔ155 in which exons 5, 6 and 11 are deleted, RON△160 in which exons 5 and 6 are deleted, or RON△165 in which exon 11 is deleted; and
providing information that the pharmaceutical composition according to claim 1 is suitable for the prevention or treatment of small cell lung cancer to a subject in which the mutation in RON is detected.

10. Use of the pharmaceutical composition according to any one of claims 1 to 7 for the prevention or treatment of small cell lung cancer.
